(19) Europäisches Patentamt
European Patent Office
Office européen des brevets

(11) EP 4 245 842 A1

(12) # EUROPEAN PATENT APPLICATION
published in accordance with Art. 153(4) EPC

(43) Date of publication:
**20.09.2023 Bulletin 2023/38**

(21) Application number: **21892340.7**

(22) Date of filing: **11.11.2021**

(51) International Patent Classification (IPC):
***C12N 1/20*** *(2006.01)* ***A01N 63/28*** *(2020.01)*
***C12R 1/465*** *(2006.01)* ***G01N 30/02*** *(2006.01)*

(52) Cooperative Patent Classification (CPC):
**A01N 63/28; C12N 1/20; G01N 30/02;**
C12R 2001/465

(86) International application number:
**PCT/KR2021/016450**

(87) International publication number:
**WO 2022/103173 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.11.2020 KR 20200153115**
**09.11.2021 KR 20210153374**

(71) Applicant: **Industry Foundation of Chonnam National University**
**Gwangju 61186 (KR)**

(72) Inventors:
- **KIM, Jin Cheol**
  **Gwangju 61105 (KR)**
- **NGUYEN, Thi Thu Hang**
  **Gwangju 61184 (KR)**
- **PARK, Ae Ran**
  **Chuncheon-si, Gangwon-do 24340 (KR)**
- **YU, Nan Hee**
  **Gwangju 61092 (KR)**

(74) Representative: **Plougmann Vingtoft a/s**
**Strandvejen 70**
**2900 Hellerup (DK)**

Remarks:
The complete document including Reference Table(s) and the Sequence Listing(s) can be downloaded from the EPO website

(54) **PLANT DISEASE CONTROL COMPOSITION COMPRISING CULTURE FLUID OF STREPTOMYCES SP. JCK-6141 STRAIN OR EXTRACT OF CULTURE FLUID OF STRAIN, PREPARATION METHOD THEREFOR AND PLANT DISEASE CONTROL METHOD**

(57) The present disclosure relates to a plant disease control composition comprising a culture broth of *Streptomyces* sp. JCK-6141 strain or an extract of the culture broth of the strain, a preparation method therefor and a plant disease control method. The culture broth of the strain or an extract thereof exhibits extremely excellent antimicrobial activity when applied to a causative pathogen of plant disease and thus may be effectively used for plant disease control.

Fig. 1

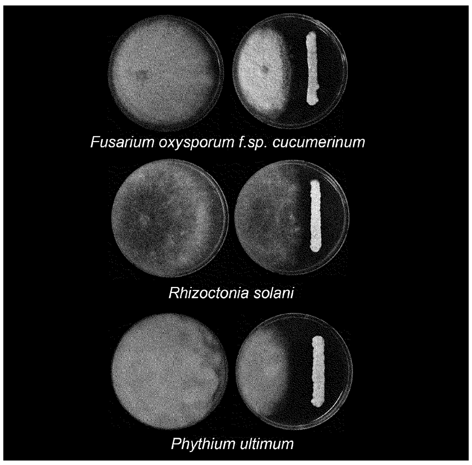

## Description

### Technical Field

The present disclosure has been made with the support of the Rural Development Administration under Project ID. No. PJ014978012020, which was conducted by the Industry Foundation of Chonnam National University in the research program named "Development of New-Concept Eco-Friendly Disinfectant Using Microbe-Derived Material" as a branch of the research project titled "Technology for Improving Agricultural Environment Using Microorganisms (R&D)" under the research management of the Rural Development Administration, from January 01, 2020 to December 31, 2020.

This application claims priority to and the benefit of Korean Application Number 10-2020-0153115, filed in the Korean Intellectual Property Office on November 16, 2020, the entire content of which is incorporated herein by reference.

This application claims priority to and the benefit of Korean Application Number 10-2021-0153374, filed in the Korean Intellectual Property Office on November 9, 2021, the entire content of which is incorporated herein by reference.

The present disclosure relates to a composition including a culture of *Streptomyces* sp. JCK-6141 strain or an extract from the culture for controlling plant diseases, a method for producing same, and a method for controlling plant diseases and, more particularly, to a disease control efficacy identified in a culture of Streptomyces sp. JCK-6141 having an antifungal activity or an extract from the culture against phytopathogens.

### Background Art

Phytopathogenic fungi settle inside or on the surface of plants to siphon nutrients from the plants and propagate, causing serious damage to various crops worldwide. Plant diseases caused by fungi are more diverse and more damaging than other pathogens, to the extent that 17 out of the 33 cases of severe damage by plant diseases in the past were caused by fungi.

In addition, ten types of phytopathogenic fungi, including *Magnaporthe oryzae, Botrytis cinerea, Puccinia* spp., *Fusarium graminearum, Fusarium oxysporum, Blumeria graminis, Mycospaerella graminicola, Colletotrichum* spp., *Ustilago maydis,* and *Melampsora lini,* have been reported to be scientifically and economically important worldwide.

Plant diseases caused by phytopathogenic fungi are very difficult to control for the following reasons. First, many phytopathogenic fungi efficiently and sustainably produce an excessively large number of spores to infect other healthy plants. Second, it takes a short time for spores to be produced in the host after infection and the mobility of spores is also good, so their infectivity is high. Third, phytopathogenic fungi have good ability to absorb nutrients from the host and sometimes secrete toxic substances or enzymes that are harmful to the host. Due to these characteristics, phytopathogenic fungi parasitize the host plant in spite of continuous control efforts, causing enormous economic damage.

For the past several decades, chemical fungicides have been used to control plant fungal diseases, but their repeated use and abuse thereof have given rise to serious problems, such as contamination of the environment including soil and surrounding water, reduction of beneficial microorganisms, residual toxicity, and occurrence of resistant strains. Accordingly, in order to solve these problems, eco-friendly biocontrol agents using microorganisms and microorganism-derived natural substances that can replace chemical pesticides have been proposed as an alternative, and related studies have been actively ongoing to date.

However, since the efficacy and type of the previously developed biocontrol agents are insufficient compared to chemical pesticides, there is an urgent need to develop better biocontrol agents.

*Streptomyces* is the largest genus of Actinomycetota. Like other Actinomycetota, *Streptomyces* is gram-positive and has genomes with a high GC content. Most *Streptomyces* found primarily in soil and decaying vegetation produce spores and are characterized by complex secondary metabolism. They are also known to produce clinically useful antibiotics of natural origin, including neomycin, cephamycin, grisemycin, bottromycin, and chloramphenicol. As various physiologically active substances as well as antibiotics are isolated from the Streptomyces spp., the strains are being treated as industrially and medically very important microorganisms. Research on these antibiotics advances from finding substances effective against bacterial or fungal infections to searching for substances effective against viral diseases, but most studies have focused on medicine.

Previous *in vitro* studies using the genus *Streptomyces* have reported the inhibitory effects of Actinomycetes against the growth of various phytopathogenic microorganisms and stated that chitinases secreted from *Streptomyces* spp. could act on the cell walls of fungi to repress the growth of fungi. Given these characteristics, the genus *Streptomyces* has excellent potential as an antimicrobial biocontrol agent.

Therefore, there is an urgent need to develop an antifungal biocontrol agent using the genus *Streptomyces.*

## Detailed Description of the Invention

### Technical Problem

Research conducted by the present inventors results in the finding that when applied to pathogens causative of plant disease, a culture of *Streptomyces* sp. JCK-6141 or an extract from the culture exhibited significantly high antifungal activity.

Accordingly, an aspect of the present disclosure is to provide a *Streptomyces* sp. JCK-6141 strain deposited with accession number KCTC 14333BP having antifungal activity.

Another aspect of the present disclosure is to provide a composition for controlling plant diseases, the composition including a culture of *Streptomyces* sp. JCK-6141 strain deposited with accession number KCTC 14333BP or an extract from the culture.

Another aspect of the present disclosure is to provide a method for preparing a composition for controlling plant diseases, which includes a culture of *Streptomyces* sp. JCK-6141 strain deposited with accession number KCTC 14333BP or an extract from the culture.

Another aspect of the present disclosure is to provide a method for preventing plant diseases, which includes steps of treating a plant with a composition containing a culture of *Streptomyces* sp. JCK-6141 strain deposited with accession number KCTC 14333BP or an extract from the culture to control plant diseases.

### Technical Solution

The present disclosure relates to a composition containing a culture of *Streptomyces* sp. JCK-6141 strain or an extract from the culture of the strain for controlling plant diseases, a preparation method therefor, and a method for controlling plant diseases. The composition for controlling plant diseases exhibits excellent antifungal activity when applied to pathogens of plant diseases.

The present inventors found that a culture of *Streptomyces* sp. JCK-6141 strain or an extract from the culture exhibited excellent antifungal activity when applied to pathogens of plant diseases.

Hereinafter, a detailed description will be given of the present disclosure.

An aspect of the present disclosure is a *Streptomyces* sp. JCK-6141 strain deposited with accession number KCTC 14333BP and having antifungal activity.

Another aspect of the present disclosure is a composition containing a Streptomyces sp. JCK-6141 strain deposited with accession number KCTC 14333BP or an extract from the culture to control plant diseases.

As used herein, the term "culture" refers to a culture medium containing microorganisms after culturing microorganisms. The term "culture supernatant" means an upper liquid obtained by removing most of microorganisms from the culture solution through centrifugation, and also referred to as "supernatant". The term "culture filtrate" is meant a liquid that is obtained by centrifugation and filtration from the culture and is free completely of microorganisms.

As used herein, the term "extract" refers to a matter extracted from a culture of the strain and is intended to encompass fractions.

In the present disclosure, plant diseases may be caused by one or more strains selected from the group consisting of fungi and oomycetes, but with no limitations thereto.

Herein, the fungus may be at least one selected from the group consisting of *Athelida rolfsii, Botryosphaeria dothidea, Botrytis cinerea, Colletotrichum caudatum, Colletotrichum cocodes, Colletotrichum horii, Curvularia lunata, Endothia parasitica, Fusarium fujikuroi, Fusarium graminearum, Fusarium oxysporum* f. sp. *raphani, Fusarium oxysporum* f. sp. *lycopersici, Fusarium oxysporum* F. sp. *cucumerinum, Gaeumannomyces graminis, Magnaporthiopsis poae, Magnapothe oryzae, Pseudocercospora circumcissa, Raffaelea quercus-mongolicae, Rhizoctonia solani, Rhizoctonia cerealis, Sclerotinia homoeocarpa,* and *Valsa cerasperma,* but is not limited thereto.

In the present disclosure, the fungus may be *Puccinia recondita,* but is not limited thereto.

In the present disclosure, *Puccinia recondita* is an obligate biotrophic fungus and parasitizes only in living plants.

In the present disclosure, the oomycete may be at least one selected from the group consisting of *Phytophthora cactorum, Phytophthora cambivora, Phytophthora capsici, Phytophthora cinnamomi, Pythium graminicola,* and *Pythium ultimum,* but is not limited thereto.

In the present disclosure, the extract from the culture of the strain may include at least one selected from the group consisting of reveromycin E, reveromycin E 1-methyl ester, and reveromycin E derivatives, but is not limited thereto.

Another aspect of the present disclosure is a method for preparing a composition for controlling plant diseases, which comprises a culturing step of preparing a culture by culturing the Streptomyces sp. JCK-6141 strain deposited with accession number KCTC 14333BP.

In the present disclosure, plant diseases may be caused by one or more strains selected from the group consisting of fungi and oomycetes, but with no limitations thereto.

In the present disclosure, the fungus may be at least one selected from the group consisting of *Athelida rolfsii, Botryosphaeria dothidea, Botrytis cinerea, Colletotrichum caudatum, Colletotrichum cocodes, Colletotrichum horii, Curvularia lunata, Endothia parasitica, Fusarium fujikuroi, Fusarium graminearum, Fusarium oxysporum* f. sp. *raphani, Fusarium oxysporum f. sp. lycopersici, Fusarium oxysporum* F. sp. *cucumerinum, Gaeumannomyces graminis, Magnaporthiopsis poae, Magnapothe oryzae, Pseudocercospora circumcissa, Raffaelea quercus-mongolicae, Rhizoctonia solani, Rhizoctonia cerealis, Sclerotinia homoeocarpa,* and *Valsa cerasperma,* but is not limited thereto.

In the present disclosure, the fungus may be *Puccinia recondita,* but is not limited thereto.

In the present disclosure, *Puccinia recondita* is an obligate biotrophic fungus and parasitizes only in living plants.

In the present disclosure, the oomycete may be at least one selected from the group consisting of *Phytophthora cactorum, Phytophthora cambivora, Phytophthora capsici, Phytophthora cinnamomi, Pythium graminicola,* and *Pythium ultimum,* but is not limited thereto.

In the present disclosure, the culturing step may include the following fractionation steps:

a first fractionation step of obtaining a first active fraction from the culture;
a second fractionation step of obtaining a second active fraction from the first active fraction using column chromatography and thin layer chromatography;
a third fractionation step of obtaining a third active fraction from the second active fraction using high-pressure liquid chromatography;
a fourth fractionation step of separating a fourth active fraction from the third active fraction using preparative thin layer chromatography; and
a pure substance identification step of identifying the fourth active fraction by high-performance liquid chromatography.

In the present disclosure, the first fractionation step may be conducted using at least one solvent selected from the group consisting of ethyl acetate, ethyl ether, ethanol, and butanol, for example, ethyl acetate, but with no limitations thereto.

In the present disclosure, the second fractionation step may include the following processes:

a concentrate preparation step of concentrating the first active fraction;
an eluate preparation step of eluting the concentrate; and
a fraction preparation step of fractionating the eluate.

In the present disclosure, the concentrate preparation step may include a process of concentrating with a reduced pressure concentrator, but with no limitations thereto.

In the eluate preparation step of the present disclosure, a silica gel column may serve as a stationary phase, but with no limitations thereto.

In the eluate preparation step of the present disclosure, at least one selected from the group consisting of chloroform, methanol, water, and acetic acid may be used as a mobile phase, but with no limitations thereto.

In the present disclosure, the fraction preparation step may be carried out by silica gel thin layer chromatography (TLC) while the eluate is loaded, but with no limitations thereto.

In the fraction preparation step of the present disclosure, at least one selected from the group consisting of chloroform, methanol, water, and acetic acid may be used as a mobile phase, but with no limitations thereto.

In the present disclosure, the fractions eluted in the second fractionation step may be used alone or in combination, but with no limitations thereto.

In the third fractionation step of the present disclosure, elution may be eluted using distilled water containing 0.1% trifluoroacetic acid: acetonitrile at a ratio of 20:80 to 50:50 or 30:70 to 50:50, but with no limitations thereto.

In the pure substance identification step of the present invention, an Xbridge column may be used as a stationary phase but with no limitations thereto.

In the pure substance identification step of the present invention, a 50 to 100% acetonitrile solution may be used as a mobile phase, but with no limitations thereto.

In the present disclosure, the pure substance may be at least one selected from the group consisting of reveromycin E, reveromycin E 1-methyl ester, and reveromycin E derivatives, but is not limited thereto.

In the present disclosure, reveromycin E has a molecular weight of 688 and a molecular formula of $C_{38}H_{56}O_{11}$.

In the present disclosure, reveromycin E has the following Chemical Formula (I):

[Chemical Formula (I)].

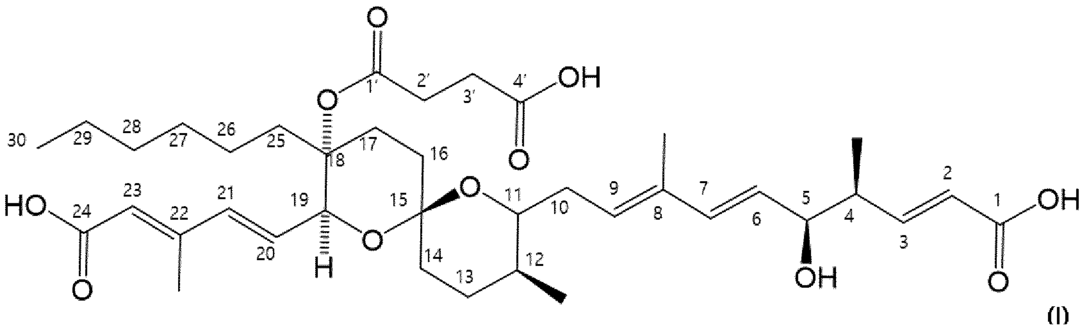

(I)

In the present disclosure, reveromycin E 1-methyl ester has a molecular weight of 702 and a molecular formula Of $C_{39}H_{58}O_{11}$.

In the present disclosure, reveromycin E 1-methyl ester has the following Chemical Formula (II):

[Chemical Formula (II)]

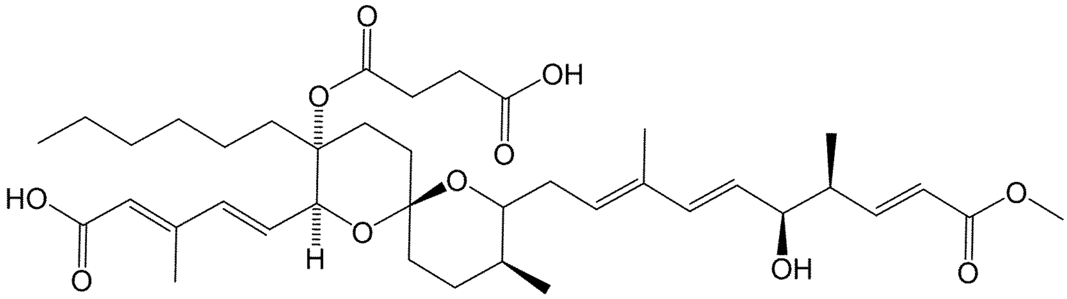

(II)

In the present disclosure, reveromycin E derivative has a molecular weight of 716 and a molecular formula of $C_{40}H_{60}O_{11}$.

Another aspect of the present disclosure is a method for controlling plant diseases, which comprises a step of treating a plant with a composition including a culture of *Streptomyces* sp. JCK-6141 strain deposited with accession number KCTC 14333BP or an extract from the culture to control plant diseases.

In the present disclosure, the plant disease may be caused by at least one selected from the group consisting of fungi and oomycetes, but with no limitations thereto.

In the present disclosure, the fungus may be at least one selected from the group consisting of *Athelida rolfsii, Botryosphaeria dothidea, Botrytis cinerea, Colletotrichum caudatum, Colletotrichum cocodes, Colletotrichum horii, Curvularia lunata, Endothia parasitica, Fusarium fujikuroi, Fusarium graminearum, Fusarium oxysporum* f. sp. *raphani, Fusarium oxysporum* f. sp. *lycopersici, Fusarium oxysporum* f. sp. *cucumerinum, Gaeumannomyces graminis, Magnaporthiopsis poae, Magnapothe oryzae, Pseudocercospora circumcissa, Raffaelea quercus-mongolicae, Rhizoctonia solani, Rhizoctonia cerealis, Sclerotinia homoeocarpa,* and *Valsa cerasperma,* but is not limited thereto.

In the present disclosure, the fungus may be *Puccinia recondita,* but is not limited thereto.

In the present disclosure, *Puccinia recondita* is an obligate biotroph fungus and parasitizes only in living plants.

In the present disclosure, the oomycete is at least one selected from the group consisting of *Phytophthora cactorum, Phytophthora cambivora, Phytophthora capsici, Phytophthora cinnamomi, Pythium graminicola,* and *Pythium ultimum,* but is not limited thereto.

In the present disclosure, the extract from the culture of the strain may include at least one selected from the group consisting of reveromycin E, reveromycin E 1-methyl ester, and reveromycin E derivatives, but is not limited thereto.

In the present disclosure, the composition-treating step may be carried out in a manner selected from the group consisting of soil drenching, soil irrigation, foliar spray, trunk injection, foliage treatment, rhizosphere treatment, and seed treatment, but with no limitations thereto.

As used herein, the term "drenching" refers to a method of injecting or spraying a drug by injecting a chemical solution into a hole dug into soil or trees.

As used herein, the term "soil drenching" is a method of injecting or spraying a chemical solution into crop

cultivation soil.

The overlapping common contents between the preparation methods for a composition for controlling plant diseases and the plant disease-controlling method composition and the composition for controlling a plant disease are omitted to avoid undue complexity of the specification.

## Advantageous Effects

The present disclosure relates to a composition containing a culture of *Streptomyces* sp. JCK-6141 strain or an extract from the culture for controlling a plant disease, a preparing method therefor, and a method for controlling a plant disease. Since they exhibit excellent antifungal activity upon application to pathogens of plant diseases, the culture of the strain and the extract from the culture can effectively control plant diseases.

## Brief Description of the Drawings

FIG. 1 is a photographic image showing the mycelial growth inhibitory effect of *Streptomyces* sp. JCK-6141 strain on phytopathogens according to an embodiment of the present disclosure.

FIG. 2 is a schematic diagram of a process of isolating and identifying reveromycin E (F61), reveromycin E 1-methyl ester (F71), and reveromycin E derivative (F81) from the *Streptomyces* sp. JCK-6141 strain culture according to an embodiment of the present disclosure.

FIG. 3A is an HPLC chromatogram of fraction F61 isolated from a culture of *Streptomyces* sp. JCK-6141 strain culture according to an embodiment of the present disclosure.

FIG. 3B is an HPLC chromatogram of fraction F71 isolated from a culture of *Streptomyces* sp. JCK-6141 strain culture according to an embodiment of the present disclosure.

FIG. 3C is an HPLC chromatogram of fraction F81 isolated from a culture of *Streptomyces* sp. JCK-6141 strain culture according to an embodiment of the present disclosure.

FIG. 4A is a negative ion-mode ESI-MS spectrum of reveromycin E (F61) isolated from a culture of *Streptomyces* sp. JCK-6141 strain according to an embodiment of the present disclosure.

FIG. 4B is a positive ion-mode ESI-MS spectrum of reveromycin E (F61) isolated from a culture of *Streptomyces* sp. JCK-6141 strain according to an embodiment of the present disclosure.

FIG. 5A is a negative ion-mode ESI-MS spectrum of reveromycin E 1-methyl ester (F71) isolated from a culture of *Streptomyces* sp. JCK-6141 strain according to an embodiment of the present disclosure.

FIG. 5B is a positive ion-mode ESI-MS spectrum of reveromycin E 1-methyl ester (F71) isolated from a culture of *Streptomyces* sp. JCK-6141 strain according to an embodiment of the present disclosure.

FIG. 6A is a negative ion-mode ESI-MS spectrum of reveromycin E (F81) isolated from a culture of *Streptomyces* sp. JCK-6141 strain according to an embodiment of the present disclosure.

FIG. 6B is a positive ion-mode ESI-MS spectrum of a reveromycin E derivative (F81) isolated from a culture of *Streptomyces* sp. JCK-6141 strain according to an embodiment of the present disclosure.

FIG. 7A shows a structural formula illustrating the chemical structure of reveromycin E (F61) isolated and identified from a culture of *Streptomyces* sp. JCK-6141 strain according to an embodiment of the present disclosure.

FIG. 7B shows a chemical structure of reveromycin E 1-methyl ester (F71) isolated and identified from a culture of *Streptomyces* sp. JCK-6141 strain according to an embodiment of the present disclosure.

FIG. 8 is a photographic image showing the inhibitory activity against 21 phytopathogenic fungi and oomycetes of an ethyl acetate fraction of a culture of *Streptomyces* sp. JCK-6141 strain according to an embodiment of the present disclosure.

FIG. 9A is a graph eludicating controlling effects against cucumber damping-off in 1 DBI treated with a culture of *Streptomyces* sp. JCK-6141 strain and an ethyl acetate fraction thereof according to an embodiment of the present disclosure.

FIG. 9B is a photographic image showing controlling effects against cucumber damping-off in 1 DBI treated with a culture of *Streptomyces* sp. JCK-6141 strain and an ethyl acetate fraction thereof according to an embodiment of the present disclosure.

FIG. 10A is a graph eludicating controlling effects against cucumber damping-off in 1 HAI treated with a culture of *Streptomyces* sp. JCK-6141 strain and an ethyl acetate fraction thereof according to an embodiment of the present disclosure.

FIG. 10B is a photographic image showing controlling effects against cucumber damping-off in 1 HAI treated with a culture of *Streptomyces* sp. JCK-6141 strain and an ethyl acetate fraction thereof according to an embodiment of the present disclosure.

FIG. 11A is a graph eludicating controlling effects against Fusarium wilt in 1 DBI treated with a culture of *Streptomyces* sp. JCK-6141 strain and an ethyl acetate fraction thereof according to an embodiment of the present disclosure.

FIG. 11B is a photographic image showing controlling effects against Fusarium wilt in 1 DBI treated with a culture of *Streptomyces* sp. JCK-6141 strain and an ethyl acetate fraction thereof according to an embodiment of the present disclosure.

FIG. 12A is a graph eludicating controlling effects against cucumber Fusarium wilt in 1 HAI treated with a culture of *Streptomyces* sp. JCK-6141 strain and an ethyl acetate fraction thereof according to an embodiment of the present disclosure.

FIG. 12B is a photographic image showing controlling effects against Fusarium wilt in 1 HAI treated with a culture of *Streptomyces* sp. JCK-6141 strain and an ethyl acetate fraction thereof according to an embodiment of the present disclosure.

FIG. 13 is a photographic image showing the controlling effects against rice sheath blight in the rice cultivar Nakdong treated with a culture of *Streptomyces* sp. JCK-6141 strain according to an embodiment of the present disclosure.

FIG. 14 is a photographic image showing the controlling effects against wheat leaf rust in the wheat cultivar Eunpa treated with a culture of *Streptomyces* sp. JCK-6141 strain according to an embodiment of the present disclosure.

FIG. 15 is a photographic image showing the controlling effects against Fusarium head blight in the wheat cultivar Eunpa treated with a culture of *Streptomyces* sp. JCK-6141 strain according to an embodiment of the present disclosure.

FIG. 16A is a graph eludicating controlling effects against dollar spot disease in creeping bentgrass treated with a culture of *Streptomyces* sp. JCK-6141 strain according to an embodiment of the present disclosure.

FIG. 16B is a photographic image showing the controlling effects against dollar spot disease in creeping bentgrass treated with a culture of *Streptomyces* sp. JCK-6141 strain according to an embodiment of the present disclosure.

## Best Mode for Carrying Out the Invention

The present disclosure relates to *Streptomyces* sp. JCK-6141 strain, deposited under accession number KCTC 14333BP, having antifungal activity.

## Mode for Carrying Out the Invention

Hereinafter, the present disclosure will be described in more detail through examples. These examples are only for illustrating the present disclosure in more detail, and it will be apparent to those skilled in the art that the scope of the present disclosure is not limited by these examples according to the gist of the present disclosure.

Unless otherwise stated, "%" used to indicate the concentration of a specific substance is (weight/weight) % for solid/solid, (weight/volume) % for solid/liquid, and (volume/volume) % for liquid/liquid throughout the specification.

EXAMPLE 1. Isolation of *Streptomyces* sp. JCK-6141 Strain

Strains with plant disease control activity were isolated from the weed rhizosphere (soil environment in which growing roots absorb and store substances and thus make various changes to the proportion of constituent organisms) in Gokseong, Jeollanam-do, South Korea. After being sampled, 1 g of soil was diluted in phosphate buffered saline (pH 7.0), spread on a tryptic soy agar (TSA) plate, and incubated for 3 to 7 days at 30°C to isolate strains.

EXAMPLE 2. Molecular Biological Analysis and Phylogenetic Analysis of *Streptomyces* sp. JCK-6141 Strain

Molecular biological identification was made of the strain isolated from the soil by sequencing 16S rRNA. The strain was inoculated into ISP2 medium and then cultured with shaking at 180 rpm at 28°C for 7 days. Then, genomic DNA (gDNA) of the strain was extracted using the I-genomic BYF DNA Extraction Mini kit (iNtRON Biotechnology, Korea) according to the protocol. Specifically, the extracted gDNA of the strain was mixed with PCR-premix (polymerase chain reaction-premix, iNtRON Biotechnology, Korea) and the primer set 9F/1512R capable of amplifying the 16S rRNA of the strain, listed Table 1 below, before PCR amplification.

TABLE 1

| SEQ ID NO: | Name | Sequence (5'---> 3') | bp |
|---|---|---|---|
| 1 | JCK-6141 16S rRNA 9F forward primer | GAGTTTGATCCTGGCTCA | 18 bp |
| 2 | JCK-6141 16S rRNA 1512R reverse primer | ACGGCTACCTTGTTACGACTT | 21 bp |

PCR started at 95°C for 5 minutes and proceeded with 30 rounds of 95°C for 30 seconds, 55°C for 30 seconds, and 72°C for 30 seconds, followed by extension at 72°C for 7 minutes and termination at 12°C.

The PCR product of 16S rRNA gene was based sequenced by Genotech (Daejeon, Korea).

The 16S rRNA encoding nucleotide sequence of the isolated JCK-6141 strain was determined to have a total nucleotide sequence of 1338 bp, and the results are shown in Table 2.

TABLE 2

| SEQ ID NO: | Name | Sequence (5'---> 3') | bp |
|---|---|---|---|
| 3 | JCK-6141 16S rRNA | GGATTAGTGG CGAACGGGTG AGTAACACGT GGGCAATCTG CCCTTCACTC TGGGACAAGC CCTGGAAACG GGGTCTAATA CCGGATATGA GCCTGGGAGG CATCTCCCGG GTTGTAAAGC TCCGGCGGTG AAGGATGAGC CCGCGGCCTA TCAGCTTGTT GGTGAGGTAA TGGCTCACCA | 1338 bp |

(continued)

| SEQ ID NO: | Name | Sequence (5'---> 3') | bp |
|---|---|---|---|
| | | AGGCGACGAC GGGTAGCCGG CCTGAGAGGG CGACCGGCCA CACTGGGACT GAGACACGGC CCAGACTCCT ACGGGAGGCA GCAGTGGGGA ATATTGCACA ATGGGCGAAA GCCTGATGCA GCGACGCCGC GTGAGGGATG ACGGCCTTCG GGTTGTAAAC CTCTTTCAGC AGGGAAGAAG CGAAAGTGAC GGTACCTGCA GAAGAAGCGC CGGCTAACTA CGTGCCAGCA GCCGCGGTAA TACGTAGGGC GCAAGCGTTG TCCGGAATTA TTGGGCGTAA AGAGCTCGTA GGCGGCTTGT CACGTCGATT GTGAAAGCCC GAGGCTTAAC CTCGGGTCTG CAGTCGATAC GGGCTAGCTA GAGTGTGGTA GGGGAGATCG GAATTCCTGG TGTAGCGGTG AAATGCGCAG ATATCAGGAG GAACACCGGT GGCGAAGGCG GATCTCTGGG CCATTACTGA CGCTGAGGAG CGAAAGCGTG GGGAGCGAAC AGGATTAGAT ACCCTGGTAG TCCACGCCGT AAACGGTGGG AACTAGGTGT TGGCGACATT CCACGTCGTC GGTGCCGCAG CTAACGCATT AAGTTCCCCG CCTGGGGAGT ACGGCCGCAA GGCTAAAACT CAAAGGAATT GACGGGGGCC CGCACAAGCG GCGGAGCATG TGGCTTAATT CGACGCAACG CGAAGAACCT TACCAAGGCT TGACATACAC CGGAAAGCAT TAGAGATAGT GCCCCCCTTG TGGTCGGTGT ACAGGTGGTG CATGGCTGTC GTCAGCTCGT GTCGTGAGAT GTTGGGTTAA GTCCCGCAAC GAGCGCAACC CTTGTTCTGT GTTGCCAGCA TGCCCTTCGG GGTGATGGGG ACTCACAGGA GACCGCCGGG GTCAACTCGG AGGAAGGTGG GGACGACGTC AAGTCATCAT GCCCCTTATG TCTTGGGCTG CACACGTGCT ACAATGGCCG GTACAATGAG CTGCGATACC GTGAGGTGGA GCGAATCTCA AAAAGCCGGT CTCAGTTCGG ATTGGGGTCT GCAACTCGAC CCCATGAAGT | |

(continued)

| SEQ ID NO: | Name | Sequence (5'---> 3') | bp |
|---|---|---|---|
| | | CGGAGTCGCT AGTAATCGCA GATCAGCATT GCTGCGGTGA ATACGTTCCC GGGCCTTGTA CACACCGCCC GTCACGTCAC GAAAGTCGGT AACACCCGAA GCCGGTGG | |

Based on the nucleotide sequence, the homology test of the nucleotide sequence was performed using the Blast program (http://www.ncbi.nlm.nhi.gov) for registered information (GenBank database). As a result,as shown in Table 3, the strain was identified as *Streptomyces* sp., named *Streptomyces* sp. JCK-6141, and was deposited with accession name KCTC 14333BP.

TABLE 3

| JCK-6141 gene to be base sequenced | Identification through NCBI BlastN analysis | Similarity (%) |
|---|---|---|
| 16S rRNA | *Streptomyces* sp. HPF1204 (LC515800) | 99.85 |
| | *Streptomyces* sp. Z2 (AF068055) | 99.85 |
| | *Streptomyces* sp. MR602 (LC381941) | 99.48 |

EXAMPLE 3. Assay for Mycelial Growth Inhibitory Activity and Enzyme Activity of *Streptomyces* sp. JCK-6141 strain Against Plant Pathogens

In order to examine the effect of the isolated and selected Streptomyces sp. JCK-6141 strain on the growth of plant pathogens, measurement was made of inhibitory activity against the growth of the three plant pathogens *Fusarium oxysporum* f. sp. *cucumerinum, Pythium ultimum,* and *Rhizoctonia solani.* Mycelial fragments of the plant pathogens obtained from cultures growing at high growth rates were inoculated into one part of PDA (Potato Dextrose Agar) medium which was separately inoculated in a straight line with Streptomyces sp. JCK-6141 at a site 2.5 cm away from the inoculated part, then incubated at 28°C. When the plant pathogens grew to the edge under monitoring, degrees of inhibition against mycelial growth of the plant pathogens by the Streptomyces sp. JCK-6141 strain were measured, and the results are shown in FIG. 1.

As shown in FIG. 1, *Streptomyces* sp. JCK-6141 strain strongly inhibited the growth of plant pathogens *Fusarium oxysporum* f. sp. *cucumerinum, Pytium ultimate,* and *Rhizoctonia solani.*

EXAMPLE 4. Measurement of Minimum Inhibitory Concentration of *Streptomyces* sp. JCK-6141 Strain against Mycelial Growth of Plant Pathogens

The separated and selected *Streptomyces* sp. JCK-6141 strain was measured for minimum inhibitory concentrations against the mycelial growth of plant pathogens. *Streptomyces* sp. JCK-6141 strain was inoculated into a Glucose-Soybean meal-Sodium chloride (GSS) broth and then cultured with shaking at 180 rpm at 28°C for 7 days. After centrifuging the culture at 4500 rpm for 20 minutes, the supernatant thus obtained was filtered through a 0.2 um sterile filter to obtain the culture filtrate (hereinafter referred to as the sample). The sample was applied at concentrations of 0.16, 0.32, 0.63, 1.25, 2.5, 5, and 10% to plant pathogens mycelia. Mycelia of the plant pathogens *Fusarium oxysporum* f. sp. *lycopersici, Pythium ultimum, Rhizoctonia solani, Botrytis cinerea,* and *Fusarium oxysporum* f. sp. *cucumerinum* were cultured stationarily at 25°C for 7 days in a PDB medium. After the concentration of the mycelia was adjusted into 500 ug/ml (w/v), the mycelia were ground using a blender (DBIHAN, HG-15D).

The ground mycelia were added in an amount of 180 μL to each well of 96-well plates to which 20 μL of the sample was dispensed to form a concentration of 10%. Then, 100 μL was taken from each well treated at a concentration of 10% and mixed with 100 μL of the ground mycelia in a neighbor well to form a concentration of 5%. The dilution was repeated in such a serial dilution manner to prepare concentrations of 0.16, 0.32, 0.63, 1.25, 2.5, 5, and 10%. After incubation for 24 hours at 25°C, degrees of mycelial growth were measured, and for each group, the experiment was performed in triplicate.

TABLE 4

| Strain | MIC values (%) | | | | |
|---|---|---|---|---|---|
| | *Fusarium oxysporum* f. sp. *lycopersici* | *Pythium ultimum* | *Rhizoctonia solani* | *Botrytis cinerea* | *Fusarium oxysporum* f. sp. *cucumerinum* |
| JCK-6141 | 0.63 | 0.31 | 0.63 | 0.31 | 0.31 |

As shown in Table 4, the culture filtrate of *Streptomyces* sp. JCK-6141 strain exhibited potent inhibitory activity against the mycelial growth of various plant pathogens such as *Fusarium oxysporum* f. sp. *lycopaceae, Pytium Ultium, Rhizoctonia solani, Botrytis cinerea,* and *Fusarium oxysporum* f. sp. *cucumerinum.* Although slightly differing in the concentration of 100% growth inhibition from one pathogen to another, the strain showed MIC values (%) of 0.31 to 0.63.

EXAMPLE 5. Isolation of Antimicrobial Active Substance from *Streptomyces* sp. JCK-6141 strain and Structural Identification thereof

5-1. Isolation of antimicrobial active substance

In order to isolate the antimicrobial active substance produced by *Streptomyces* sp. JCK-6141 strain, the strain was inoculated in a Glucose-Soybean meal-Sodium chloride (GSS) broth and then cultured at 28°C for 7 days with shaking at 180 rpm. Thereafter, a supernatant was obtained by briefly spinning the culture medium (total 3 L) at 400 rpm for 10 minutes, as shown in FIG. 2. The supernatant was adjusted to a pH of 2 and fractionated twice with ethyl acetate (3 L) to prepare an ethyl acetate fraction (first active fraction) of *Streptomyces* sp. JCK-6141 strain culture.

The prepared ethyl acetate fraction (first active fraction) was concentrated using a vacuum concentrator. For further purifying antimicrobial active substances, silica gel column chromatography was performed. In this regard, the fraction was loaded into a silica gel column (500g, 200-400 mesh), followed by elution with chloroform: methanol (8:2, 1L), chloroform:methanol (7:3, 1L), and chloroform:methanol:water:acetic acid (30:9: 1:0.25, 2L). Drops of the eluted fractions were loaded to the silica gel plate for thin layer chromatography (0.25 mm film thickness) and then developed with chloroform: methanol: water:acetic acid (30:9:1:0.25). Absorbance was read at 254 nm. After para-anisaldehyde reagent was sprayed thereto, fractions with similar color development were collected and concentrated under reduced pressure.

Among the fractions concentrated under reduced pressure, one fraction showing antifungal activity against Rhizoctonia solani (second active fraction) was selected. Substances were separated from the fraction using Atlantis® T3f prep OBDTM (5 um, 19 × 250 mm) column-equipped preparative high-performance liquid chromatography (Waters, Japan). For this, 0.1% trifluoroacetic acid (TFA)-added distilled water and acetonitrile were used as an eluent. Distilled water containing 0.1% trifluoroacetic acid was added to acetonitrile to prepare 50% to 70% acetonitrile solutions. Elution was performed with 50% to 70% acetonitrile solutions at a flow rate of 15 ml/min for 50 minutes. As a result of elution, a total of 14 fractions (F1 to F14, the third active fraction) were obtained.

Among them, the three fractions F6, F7, and F8 that exhibited antifungal activity were fractioned by preparative thin layer chromatography (silica gel thin layer chromatography, 0.5 mm film thickness). Chloroform:methanol:water:acetic acid (30:9:1:0.25) was used as the developing solvent. As a result, active F61 (35.2 mg), F71 (15.3 mg), and F81 (9.2 mg) (fourth activity) fraction) were obtained.

Active pure substances were identified in F61, F71, and F81 thus obtained. In this regard, high-performance liquid chromatography was performed while the amount of organic solvent was started from 50% acetonitrile solution on Xbridge C18 (4.6 × 250 mm, 5 um) column, increasing to 100% acetonitrile solution in 35 minutes. Active substances were detected by a PDA (photodiode array) detector at a detection wavelength of 240 nm. The results are shown in FIGS. 3A to 3C. In particular, F71 was observed to have two isomers or almost similar substances in a mixture at a ratio of 1:1.

5-2. Molecular weight identification of active antimicrobial substances

The three substances F61, F71, and F81 isolated from *Streptomyces* sp. JCK-6141 strain were lyophilized and measured for molecular weights through liquid chromatography electrospray mass method (LC-ESI-MS, API2000, ABSCIEX, USA) in negative and positive ion modes, and the results are depicted in FIGS. 4A to 6B.

As shown in FIGS. 4A and 4B, [M-1]- and [M+Na]+ ion peaks of F61 appeared at m/z 687.3749 in negative ion mode and 711.3725 in positive ion mode, respectively, giving the molecular formula $C_{38}H_{56}O_{11}$ with a molecular weight of 688. In the case of F71, as shown in FIGS. 5A and 5B, ion peaks were observed at m/z 701.3885 for [M-1]- in negative ion mode and at m/z 725.3878 for [M + Na]+ in positive ion mode, giving the molecular formula $C_{39}H_{58}O_{11}$ with a

molecular weight of 702. In addition, as shown in FIGS. 6A and 6B, ion peaks of F81 appeared at m/z 715.4095 for [M-1]- ion in negative ion mode and at m/z 739.4048 for [M+Na]+ in positive ion mode, indicating the molecular formula of $C_{40}H_{60}O_{11}$ with a molecular weight of 716. The compounds F71 and F81 were estimated to be reveromycin E derivatives as their molecular weights showed a difference of 14 and 28, respectively, when compared to F61.

5-3. Chemical structures of active antifungal substances

In order to identify the chemical structures of F61 and F71, various modes of NMR analysis were performed. Among them, NMR data for F61 are summarized in Table 5 below.

TABLE 5

| Position | H | C | COSY | HMBC |
|---|---|---|---|---|
| 1 | | 168.87 | | 2, 3, 1-ME |
| 2 | 5.82 (dt) | 121.25 | 3, 4 | 1, 4, 5, 1-ME |
| 3 | 6.98 (dd) | 151.27 | 2, 4 | 1, 2, 4, 5, 4-Me |
| 4 | 2.53 (q) | 42.89 | 3, 5 | 2, 3, 5, 4-Me |
| 5 | 4.07 (q) | 75.68 | 4, 6, 7, 4-Me | 4, 6, 7, 4-Me |
| 6 | 5.52 (m) | 126.52 | 4, 5, 7 | 5, 7, 8 |
| 7 | 6.25 (dd) | 136.3 | 5, 6 | 5, 6, 8, 8-Me |
| 8 | | 140.36 | | 9, 10, 8-Me |
| 9 | 5.55 (d) | 126.41 | 7, 10a | 8, 10, 8-Me |
| 10a | 2.38 (dt) | 31.57 | 9, 11 | 8, 9, 11, 8-Me |
| 10b | 1.83 (d) | | | |
| 11 | 3.46 (dt) | 74.87 | 10a, 12 | 9, 10 |
| 12 | 1.42 (dd) | 33.38 | 11, 13,12-Me | 10a, 11, 13 |
| 13a | 1.48 (m) | 27.37 | 14a | 11, 12, 14, 15 |
| 13b | 1.53 (dd) | | | |
| 14a | 1.74 (d) | 35.5 | 13a | |
| 14b | 1.45 (d) | | | 13, 15, 16 |
| 15 | | 95.66 | | 14, 16, 17 |
| 16a | 1.83 (d) | 31.36 | 17 | 14,15,17,18 |
| 16b | 1.60 (d) | | 17 | 15, 17, 18 |
| 17a | 2.02 (m) | 24.04 | 16ab, 17b | 15 |
| 17b | 2.34 (t) | | 16ab, 19 | 18, 19, 20 |
| 18 | | 82.85 | | 16, 17b, 25 |
| 19 | 4.61 (d) | 78.37 | 17b, 20 | 15, 17, 18, 20, 21 |
| 20 | 6.44 (s) | 132.69 | 19 | 19, 21 |
| 21 | 6.46 (s) | 137.86 | | 22 |
| 22 | | 150.95 | | 21, 23 |
| 23 | 5.88 (d) | 120.41 | 22-Me | 21, 22, 24, 22-Me |
| 24 | | 168.77 | | 22-Me,23 |
| 25a | 1.87 (d) | 34.17 | 26 | 18, 26 |
| 25b | 1.61 (d) | | 26 | |

(continued)

| Position | H | C | COSY | HMBC |
|---|---|---|---|---|
| 26 | 1.24 (m) | 29.02 | 25, 27, 28 | 25, 27 |
| 27a | 1.23 (m) | 22.16 | 26, 28 | 26, 28, 29, 30 |
| 27b | 1.27 (m) | | 26, 28 | 26, 28, 29, 30 |
| 28a | 1.24 (m) | 31.2 | | 27, 29 30 |
| 28b | 1.22 (m) | | | 27, 29, 30 |
| 29a | 1.27 (m) | 21.31 | | 27, 28, 30 |
| 29b | 1.23 (m) | | | 27, 28, 30 |
| 30 | 0.86 (t) | 13.00 | 29a | 27, 28, 29 |
| 4-me | 1.09 (dd) | 13.99 | 4 | 3, 4, 5 |
| 8-me | 1.76 (s) | 11.61 | 6 | 7, 8, 9 |
| 12-me | 0.79 (t) | 16.69 | 12 | 11, 12, 13 |
| 22-me | 2.27 (s) | 13.27 | 21, 23 | 20, 21, 22, 23, 24 |
| 1’ | | 171.97 | | 2’, 3’ |
| 2’ | 2.61 (m) | 29.76 | | 1’, 3’, 4’ |
| 3’ | 2.58 (t) | 28.51 | | 2’ , 3’ , 4’ |
| 4’ | | 174.54 | | 2’, 3’ |
| 1-ME | 2.27 (s) | 51.05 | 2 | 1, 2 |

As shown from the data of Table 5 and FIG. 7A, the chemical structure of F61 was identified as reveromycin E.

NMR data of F71 are summarized in Table 6 below.

TABLE 6

| position | H | C | COSY | HMBC |
|---|---|---|---|---|
| 1 | | 168.87 | | 2, 3, 1-ME |
| 2 | 5.82 (dt) | 121.25 | 3, 4 | 1, 4, 5, 1-ME |
| 3 | 6.98 (dd) | 151.27 | 2, 4 | 1, 2, 4, 5, 4-Me |
| 4 | 2.53 (q) | 42.89 | 3, 5 | 2, 3, 5, 4-Me |
| 5 | 4.07 (q) | 75.68 | 4, 6, 7, 4-Me | 4, 6, 7, 4-Me |
| 6 | 5.52 (m) | 126.52 | 4, 5, 7 | 5, 7, 8 |
| 7 | 6.25 (dd) | 136.3 | 5, 6 | 5, 6, 8, 8-Me |
| 8 | | 140.36 | | 9, 10, 8-Me |
| 9 | 5.55 (d) | 126.41 | 7, 10a | 8, 10, 8-Me |
| 10a | 2.38 (dt) | 31.57 | 9, 11 | 8, 9, 11, 8-Me |
| 10b | 1.83 (d) | | | |
| 11 | 3.46 (dt) | 74.87 | 10a, 12 | 9, 10 |
| 12 | 1.42 (dd) | 33.38 | 11, 13,12-Me | 10a, 11, 13 |
| 13a | 1.48 (m) | 27.37 | 14a | 11, 12, 14, 15 |
| 13b | 1.53 (dd) | | | |
| 14a | 1.74 (d) | 35.5 | 13a | |

(continued)

| position | H | C | COSY | HMBC |
|---|---|---|---|---|
| 14b | 1.45 (d) | | | 13, 15, 16 |
| 15 | | 95.66 | | 14, 16, 17 |
| 16a | 1.83 (d) | 31.36 | 17 | 14,15,17,18 |
| 16b | 1.60 (d) | | 17 | 15, 17, 18 |
| 17a | 2.02 (m) | 24.04 | 16ab, 17b | 15 |
| 17b | 2.34 (t) | | 16ab, 19 | 18, 19, 20 |
| 18 | | 82.85 | | 16, 17b, 25 |
| 19 | 4.61 (d) | 78.37 | 17b, 20 | 15, 17, 18, 20, 21 |
| 20 | 6.44 (s) | 132.69 | 19 | 19, 21 |
| 21 | 6.46 (s) | 137.86 | | 22 |
| 22 | | 150.95 | | 21, 23 |
| 23 | 5.88 (d) | 120.41 | 22-Me | 21, 22, 24, 22-Me |
| 24 | | 168.77 | | 22-Me,23 |
| 25a | 1.87 (d) | 34.17 | 26 | 18, 26 |
| 25b | 1.61 (d) | | 26 | |
| 26 | 1.24 (m) | 29.02 | 25, 27, 28 | 25, 27 |
| 27a | 1.23 (m) | 22.16 | 26, 28 | 26, 28, 29, 30 |
| 27b | 1.27 (m) | | 26, 28 | 26, 28, 29, 30 |
| 28a | 1.24 (m) | 31.2 | | 27, 29 30 |
| 28b | 1.22 (m) | | | 27, 29, 30 |
| 29a | 1.27 (m) | 21.31 | | 27, 28, 30 |
| 29b | 1.23 (m) | | | 27, 28, 30 |
| 30 | 0.86 (t) | 13.00 | 29a | 27, 28, 29 |
| 4-me | 1.09 (dd) | 13.99 | 4 | 3, 4, 5 |
| 8-me | 1.76 (s) | 11.61 | 6 | 7, 8, 9 |
| 12-me | 0.79 (t) | 16.69 | 12 | 11, 12, 13 |
| 22-me | 2.27 (s) | 13.27 | 21, 23 | 20, 21, 22, 23, 24 |
| 1’ | | 171.97 | | 2’, 3’ |
| 2’ | 2.61 (m) | 29.76 | | 1’, 3’, 4’ |
| 3’ | 2.58 (t) | 28.51 | | 2’, 3’, 4’ |
| 4’ | | 174.54 | | 2’, 3’ |
| 1-ME | 2.27 (s) | 51.05 | 2 | 1, 2 |

As shown in Table 6 and FIG. 7B, the molecular weight and formula of F71 were the same as those of the previously known reveromycin E 4’-methyl ester, but the NMR data analysis of various modes indicated that it is reveromycin E 1-methyl ester, which has a methyl group attached to the 1-COOH group. This is the first reported substance.

EXAMPLE 6. *In Vitro* Assay for Antifungal Activity of *Streptomyces* sp. JCK-6141 Ethyl Acetate Fraction against Various Plant Pathogens

6-1. Pour-plating method

A pour-plating method was used to observe the inhibitory effect of *Streptomyces* sp. JCK-6141 strain culture containing the active antifungal substance reveromycin E against the mycelial growth of 21 species of plant pathogenic fungi (16 species) and oocytes (5 species). Briefly, PDA (potato dextrose agar) plates were treated with predetermined concentrations (0 pg/ml, 1 pg/ml, and 5 pg/ml) of the ethyl acetate fraction of a Streptomyces sp. JCK-6141 strain culture. The centers of the plates were inoculated with the pathogenic fungi and oomycetes listed in Table 7, including *Athelida rolfsii, Botryoshpaeria dothidea, Colletotrichum caudatum, Colletotrichum cocodes, Colletotrichum horii, Curvularia lunata, Endothia parasitica, Fusarium fugikuroi, Fusarium graminearum, Gaeumannomyces graminis, Pseudocerocospora circumcissa, Raffaelea quercus-mongolicae, Rhizoctonia solani, Rhizoctonia cerealis, Sclerotinia homoeocarpa, Valsa ceratosperma, Phytophthora capsici, Phytophthora cambivora, Phytophthora cinnamomi, Phythium graminicola,* and *Phythium ultimum,* which were then incubated at 25°C under the monitoring of mycelial growth.

TABLE 7

| No. | Type | Strain Name |
|---|---|---|
| 1 | Fungus | *Athelida rolfsii* |
| 2 | Fungus | *Botryoshpaeria dothidea* |
| 3 | Fungus | *Colletotrichum caudatum* |
| 4 | Fungus | *Colletotrichum cocodes* |
| 5 | Fungus | *Colletotrichum horii* |
| 6 | Fungus | *Curvularia lunata* |
| 7 | Fungus | *Endothia parasitica* |
| 8 | Fungus | *Fusarium fugikuroi* |
| 9 | Fungus | *Fusarium graminearum* |
| 10 | Fungus | *Gaeumannomyces graminis* |
| 11 | Fungus | *Pseudocerocospora circumcissa* |
| 12 | Fungus | *Raffaelea quercus-mongolicae* |
| 13 | Fungus | *Rhizoctonia solani* |
| 14 | Fungus | *Rhizoctonia cerealis* |
| 15 | Fungus | *Sclerotinia homoeocarpa* |
| 16 | Fungus | *Valsa ceratosperma* |
| 17 | Oomycete | *Phytophthora capsici* |
| 18 | Oomycete | *Phytophthora cambivora* |
| 19 | Oomycete | *Phytophthora cinnamomi* |
| 20 | Oomycete | *Phythium graminicola* |
| 21 | Oomycete | *Phythium ultimum* |

As shown in FIG. 8, the ethyl acetate fraction of *Streptomyces* sp. JCK-6141 strain culture had the effect of inhibiting the mycelial growth of the plant pathogenic fungi and oomycetes listed in Table 7. In particular, the acetate fraction of *Streptomyces* sp. JCK-6141 strain culture inhibited the mycelial growth of all phytopathogenic fungi and oomycetes in the case of 5 ug/ml treatment group. These data indicate that the ethyl acetate fraction of *Streptomyces* sp. JCK-6141 strain culture exhibited excellent antifungal activity.

6-2. 96-Well microplate method

The minimum inhibitory concentration of active antifungal substance reveromycin E (F61) ($C_{38}H_{56}O_{11}$), which

was isolated and identified in Example 5, was measured against the mycelial growth of phytopathogenic fungi. First, reveromycin E (F61) was applied at a concentration of 0.0244 to 3.125 ug/ml to mycelia of the phytopathogens listed in Table 8, including *Botryoshpaeria dothidea, Botryritis cinerea, Colletotrichum horii, Curvularia lunata, Endothia parasitica, Fusarium graminearum, Fusarium oxysporum* f. sp. *raphanin, Fusarium oxysporum* f. sp. *cucumerinum, Gaeumannomyces graminis, Magnaporthiopsis poae, Magnapothe oryzae, Phythium ultimum, Rhizoctonia solani, Sclerotinia homoeocarpa, Valsa ceratosperma, Phytophthora cactorum, Phytophthora cambivora,* and *Phytophthora cinnamomi.*

TABLE 8

| No. | Type | Strain Name |
|---|---|---|
| 1 | Fungus | *Botryoshpaeria dothidea* |
| 2 | Fungus | *Botryritis cinerea* |
| 3 | Fungus | *Colletotrichum horii* |
| 4 | Fungus | *Curvularia lunata* |
| 5 | Fungus | *Endothia parasitica* |
| 6 | Fungus | *Fusarium graminearum* |
| 7 | Fungus | *Fusarium oxysporum* f. sp. *raphanin* |
| 8 | Fungus | *Fusarium oxysporum* f. sp. *cucumerinum* |
| 9 | Fungus | *Gaeumannomyces graminis* |
| 10 | Fungus | *Magnaporthiopsis poae* |
| 11 | Fungus | *Magnapothe oryzae* |
| 12 | Fungus | *Phythium ultimum* |
| 13 | Fungus | *Rhizoctonia solani* |
| 14 | Fungus | *Sclerotinia homoeocarpa* |
| 15 | Fungus | *Valsa ceratosperma* |
| 16 | Oomycete | *Phytophthora cactorum* |
| 17 | Oomycete | *Phytophthora cambivora* |
| 18 | Oomycete | *Phytophthora cinnamomi* |

The strains listed in Table 8 were stationarily cultured at 25°C for 7 days in PDA (Potato Dextrose Agar) plates. The mycelia thus obtained were adjusted into a concentration of 500 μg/ml (w/v) and then ground using a blender (DBIHAN, HG-15D).

The ground mycelia were added 180 μl to each well of 96-well plates, and then 20 ul of the sample was dispensed to form a concentration of 3.125 pg/ml. Subsequently, 100 μL taken from the well where a concentration of 3.125 pg/ml had been prepared was mixed with 100 ul of the ground mycelia in a neighbor well to form a concentration of 1.56 ug/ml. The dilution was repeated in such a serial manner to prepare concentrations of 0.78 to 0.0224 pg/ml. After incubation for 24 hours at 25°C, degrees of mycelial growth were measured, and for each group, the experiment was performed in triplicate. The results are shown in Table 9 below.

TABLE 9

| No. | Type | Strain Name | F61 (pg/ml) |
|---|---|---|---|
| 1 | Fungus | *Botryoshpaeria dothidea* | 0.098 |
| 2 | Fungus | *Botryritis cinerea* | 1.56 |
| 3 | Fungus | *Colletotrichum horii* | 0.049 |
| 4 | Fungus | *Curvularia lunata* | 0.78 |
| 5 | Fungus | *Endothia parasitica* | 1.56 |
| 6 | Fungus | *Fusarium graminearum* | 0.195 |

(continued)

| No. | Type | Strain Name | F61 (pg/ml) |
|---|---|---|---|
| 7 | Fungus | *Fusarium oxysporum* f. sp. *raphani* | 1.56 |
| 8 | Fungus | *Fusarium oxysporum* f. sp. | 1.56 |
| | | *cucumerinum* | |
| 9 | Fungus | *Gaeumannomyces graminis* | 3.125 |
| 10 | Fungus | *Magnaporthiopsis poae* | 0.0244 |
| 11 | Fungus | *Magnapothe oryzae* | 0.78 |
| 12 | Fungus | *Phythium ultimum* | 1.56 |
| 13 | Fungus | *Rhizoctonia solani* | 1.56 |
| 14 | Fungus | *Sclerotinia homoeocarpa* | 0.195 |
| 15 | Fungus | *Valsa ceratosperma* | 0.78 |
| 16 | Oomycete | *Phytophthora cactorum* | 0.195 |
| 17 | Oomycete | *Phytophthora cambivora* | 0.195 |
| 18 | Oomycete | *Phytophthora cinnamomi* | 0.39 |

As shown in Table 9, reveromycin E (F61) exhibited potent antifungal activity to inhibit the mycelial growth of various plant pathogens. Although slightly differing in the concentration of 100% growth inhibition from one pathogen to another, reveromycin E exhibited excellent antifungal activity against the pathogens, ranging in MIC value from 0.0224 to 3.125 ug/ml. In particular, the substance exhibits excellent antifungal activity against the oomycetes *Phytophthora cactorum, Phytophthora cambivora* and *Phytophthora cinnamomi* for which MIC values were measured to range from 0.195 to 0.39 pg/ml.

Usually, Fusarium strains are more resistant to chemicals than other species. However, reveromycine E (F61) exhibited very high antifungal activity against fungi such as *Fusarium graminearum* and *Fusarium* graminearum, with MIC values therefor ranging from 1.56 and 0.195 μg/ml, respectively.

EXAMPLE 7. Assay for Lytic Enzyme Activity of *Streptomyces* sp. JCK-6141 Strain Culture

In order to assay the lytic enzyme activity of *Streptomyces* sp. JCK-6141 strain culture, the activity of the lytic enzymes protease, chitinase, and cellulase were measured. Strains having lytic enzyme activity are known to be high in chitinase activity. This characteristic confirms inhibitory activity against fungi.

For use in an assay for protease activity, agar was added to 1 % skim milk to make a plate onto which Streptomyces sp. JCK-6141 strain was then inoculated. After inoculation, the plate was stationarily incubated at 30°C. Two days after incubation, the clear zone by protease activity was observed. For chitinase activity, *Streptomyces* sp. JCK-6141 strain was inoculated onto PDA (Potato Dextrose Agar) plates containing 1.5% colloidal chitin and then stationarily incubated at 28°C for one week to create a clear zone thereon. Cellulase enzyme activity was measured by culturing *Streptomyces* sp. JCK-6141 strain in Mendel-Leeds agar plates using carboxymethyl cellulose (CMC) as the sole carbon source. Plate containing 0.4 g/L $KH_2PO_4$, 0.02 g/L $CaCl_2$, 0.02 g/L NaCl, 0.02 g/L $FeSO_47H_2O$, 2.5 g/L CMC, and 15.0 g/L agar was adjusted with 1 M NaOH to have a pH of 7.2. *Streptomyces* sp. JCK-6141 strain was inoculated onto the plate and stationarily incubated 28°C for one week while cellulase release was monitored. The activity of each protease, chitinase, and cellulase was measured, and the results are summarized in Table 10 below.

TABLE 10

| Sample | Protease activity | Chitinase Activity | Cellulase activity |
|---|---|---|---|
| JCK-6141 | - | + | +++ |

As shown in Table 10, it was confirmed that *Streptomyces* sp. JCK-6141 strain not only had chitinase activity, but, in particular, possessed excellent cellulase activity. Oomycete cell walls contain cellulose, and other fungi contain chitin as a cell wall component. Therefore, through the measurement of cellulase and chitinase activities of *Streptomyces* sp. JCK-6141 strain, it can be revealed whether *Streptomyces* sp. JCK-6141 strain has higher antimicrobial activity against oomycetes or fungi. Previously, the results of Example 6-2 demonstrated that treatment with reveromycin E

(F61) had higher inhibitory activity against mycelial growth of phytopathogenic oomycetes than phytopathogenic fungi. As such, the culture of *Streptomyces* sp. JCK-6141 strain possesses chitinase and cellulase activity as well as the low-molecular weight active antimicrobial material reveromycin E (F61), thereby exhibiting excellent antimicrobial activity against various phytopathogenic fungi and oocytes.

EXAMPLE 8. *In Vivo* Assay for Controlling Effect of Streptomyces sp. JCK-6141 Strain on Damping-Off in Cucumber

8-1. Plant preparation

"Nebakja" cucumber seeds (Syngenta, Korea), a sensitive cultivar of cucumber, were germinated for two days at 25°C on filter paper (Petri dish with 9 cm diameter) soaked in sterile water and then grown for 5 days to prepare plants (5-day-old cucumber seedlings), performing a pot experiment for an *in vivo* biocontrol of damping-off in cucumbers by JCK-6141 strain.

8-2. Sample application and inoculation

A total of four samples to be applied to the plants by soil drenching were prepared by diluting *Streptomyces* sp. JCK-6141 strain culture 100-fold and 10-fold with distilled water and fractionating the dilutions with ethyl acetate to final concentrations of 50 μg/ml and 200 ug/ml.

*Rhizoctonia solani* (hereinafter referred to as "Rs"), which causes damping-off in cucumbers, was prepared as an inoculum to be inoculated into the plant. Agar covered with active-grown Rs mycelia was cut and inoculated into a 250 ml Erlenmeyer flask containing 100 mL of PDB (potato dextrose broth). After incubation of the flask at 28°C for 4 days with shaking at 150 rpm, the cultured mycelia were filtered through 4-layer gauze and washed with sterile water. The soil for Rs mycelial inoculation was prepared by mixing 0.75 g of Rs mycelia per 1 kg of the gardening soil mix.

Five days after the germination, the soil in which cucumber seedlings were planted was drenched with 10 ml of each of the four samples. After one day of soil drenching, the soil for Rs mycelium inoculation was aliquoted into 150 g per pot and inoculated with cucumber seedlings to prepare 1 DBI. 1 DBI could reveal the preventive effect of *Streptomyces* sp. JCK-6141 on damping-off in cucumbers because the sample was pre-treated before inoculation.

The soil for Rs mycelium inoculation was aliquoted into 150 g per pot. After 5 days of germination, cucumber seedlings were transplanted, and after 1 hour, 1 HAI was prepared by drenching the seedlings with 10 ml of each of the four samples. 1 HAI can confirm the therapeutic effect because the sample was drenched after transplanting the seedlings into the soil mixed with Rs mycelium.

For a non-treated group, a solution added with Tween 20 (concentration 250 μg/ml) was used, and as a control drug, a dispersive solution of Janrokend (active ingredient Hymexazole 30%, Penthiopyrad 5%, Korea Ginseng Corporation, Korea) was diluted 1000-fold and applied. All treatments were carried out in a soil drenching manner with 10 ml per pot.

8-3. *In vivo* bioassay

Cucumber seedlings transplanted into Rs mycelium-inoculated pots were grown at 25°C for 7 days and then harvested to count diseased plants. Each treatment was repeated with triple replicates, and a replicate consisted of 10 pots. The experiment was conducted with two replicates to verify the efficacy. The disease control value was calculated using Equation 1 below, and the results of 1 DBI and 1 HAI are summarized in Tables 11 and 12, below.

[Equation 1]

$$\text{Control value (\%)} = \left(1 - \frac{\text{Disease incidence of treated group}}{\text{Disease incidence of non-treated group}}\right) \times 100$$

TABLE 11

| Treated group (1 DBI) | Concentration (fold, μg/ml ) | Control value (%) |
|---|---|---|
| CB (diluted in distilled water) | 100-fold | 96.2±8.3 |
| | 10-fold | 92.0116.6 |

(continued)

| Treated group (1 DBI) | Concentration (fold, μg/ml ) | Control value (%) |
|---|---|---|
| EtOAc ext | 50 μg/ml | 0.010.0 |
| | 200 μg/ml | 50.3157.7 |
| Janrokend | 1000-fold | 10010.0 |

TABLE 12

| Treated group (1 HAI) | Concentration (fold, μg/ml ) | Control value (%) |
|---|---|---|
| CB (diluted in distilled water) | 100-fold | 75.0150.0 |
| | 10-fold | 10010.0 |
| EtOAc Ext | 50 μg/ml | 75.0150.0 |
| | 200 μg/ml | 10010.0 |
| Janrokend | 1000-fold | 10010.0 |

As shown in Tables 11 and 12, and FIGS. 9A to 10B, the culture of *Streptomyces* sp. JCK-6141 strain reduced the incidence of Rs-caused damping-off in cucumbers, and the 100-fold and 10-fold dilutions thereof exhibited control effects similar to that of the control drug Janrokend irrespective of the sample application time. In particular, the application of a 10-fold dilution of the culture gave a control value of 100% for 1 HAI, which was similar measurement to the control value using the control agent. The ethyl acetate fraction showed control activity in a dose-dependent manner. Specifically, when the ethyl acetate fraction was applied in amounts of 50 μg/ml and 200 ug/ml to 1 HAI, the control values were about 75.0% and 100%, respectively. The data indicated that the diluted culture and ethyl acetate fractions of the culture of *Streptomyces* sp. JCK-6141 strain had excellent antifungal activity against *Rhizoctonia solani,* a causative pathogen of cucumbers damping-off.

EXAMPLE 9. *In Vivo* Assay for Controlling Effect of *Streptomyces* sp. JCK-6141 Strain on Fusarium Wilt in Cucumber

9-1. Plant preparation

The control efficacy of *Streptomyces* sp. JCK-6141 strain against Fusarium wilt was evaluated *in vivo.* Horticultural gardening soil No. 5 (Punong Sangtosa, Korea) was put into 8×8 pots (15 ml/pot, Bumnong Co. Ltd., Korea), and one seed of the cucumber species "Jungboksamcheok" (Syngenta, Korea) was sown per pot and cultivated in a greenhouse for 7 days to prepare plants to be used for the control effect evaluation.

9-2. Sample treatment

Fusarium *oxyspoirum* f. sp. *cucumerinum* (Foc) was inoculated into PDA plates and cultured at 25°C for 7 days. From the cultured plate, mycelial fragments of Foc were removed and then were inoculated into V8 broth (V-8 juice 200 ml, $CaCO_3$ 3 g, distilled water 1 L) and cultured at 25°C in a dark condition for 7 days with shaking at 150 rpm. After shaking the culture, the pathogen culture was harvested and filtered through quadruple gauze to remove mycelia, followed by centrifugation (5000 rpm, 10 minutes, 4°C). The spore pellet obtained by removing the supernatant was added with sterile water and suspended. For use as an inoculum, the Foc spore suspension was diluted with sterile water under an optical microscope to give a spore (conidium) concentration of $1.0\times10^7$ conidia/ml as countered by a hemacytometer.

9-3. *In vivo* bioassay

After being transplanted at a density of one seed per pot, pregerminated cucumber seeds were grown at 25°C into cucumber seedlings which were then treated with the sample in three ways 5 days later. The culture of *Streptomyces* sp. JCK-6141 strain was diluted 100-fold and 10-fold with distilled water, and fractionated with ethyl acetate to final concentrations of 50 μg/ml and 200 μg/ml to prepare a total of four samples.

The each samples prepared from *Streptomyces* sp. JCK-6141 strain was applied in 10 ml to 5 days-old cucumber seedlings through soil drenching. One day after the soil drenching treatment of *Streptomyces* sp. JCK-6141 strain sample, the treated group was inoculated with 10 ml of the spore suspension of the Foc strain to prepare 1 DBI. In addition, 5 days-old cucumber seedlings were inoculated with 10 ml of the spore suspension of Foc strain by soil drench. One hour after inoculation, 10 ml of the *Streptomyces* sp. JCK-6141 strain sample was applied to seedlings by soil drenching to prepare 1 HAI.

For a non-treated group, a solution added with Tween 20 (concentration 250 μg/ml) was applied, and as a control drug, a dispersive solution of Janrokend (active ingredient Hymexazole 30%, Penthiopyrad 5%, Korea Ginseng Corporation, Korea) was diluted 1000-fold and applied. All treatments were carried out in soil drenching manner with 10 ml per pot.

The inoculated cucumber seedlings were cultured in a humid room at 25°C for 24 hours, then transferred to a constant temperature room of 25°C, irradiated with light for 12 hours a day, and grown for 3 weeks. Each treatment was repeated with triple replicates, and a replicate consisted of 10 pots. The experiment was conducted with two replicates to verify the efficacy of the treatment. The disease control value was calculated using Equation 1 and the results are summarized in Tables 13 and 14, below.

TABLE 13

| Treated group (1 DBI) | Concentration (fold, μg/ml ) | Control value (%) |
|---|---|---|
| CB | 100-fold | 40.8113.2 |
| | 10-fold | 67.1113.2 |
| EtOAc Ext | 50 μg/ml | 100.010.0 |
| | 200 μg/ml | 28.6135.0 |
| Janrokend | 1000 fold | 100.010.0 |

TABLE 14

| Treated group (1 HAI) | Concentration (fold, μg/ml ) | Control value (%) |
|---|---|---|
| CB | 100-fold | 19.7119.9 |
| | 10-fold | 35.5146.0 |
| EtOAc Ext | 50 μg/ml | 40.8113.2 |
| | 200 μg/ml | 23.7127.3 |
| Janrokend | 1000-fold | 42.1143.2 |

As shown in Tables 13 and 14, and FIGS. 11A to 12B, the *Streptomyces* sp. JCK-6141 strain samples reduced the incidence of Foc-caused Fusarium wilt in cucumbers and exhibited control efficacy similar to or higher than that of the control drug Janrokend irrespective of the sample application time. In particular, the application of 50 pg/ml of the ethylacetate fraction gave a control value of 100% for 1 DBI, exhibiting the highest control performance. In addition, when applied to 1 DBI and 1 HAI, the ethylacetate fraction showed higher control activity at a low concentration (50 pg/ml) than at a high concentration (200 pg/ml), indicating that Fusarium wilt can be effectively controlled not only by the direct antifungal activity of the reveromycin substances but also by inducible resistance mechanism of these or other substances.

EXAMPLE 10. *In Vivo* Assay for Controlling Effect of Streptomyces sp. JCK-6141 Strain on Rice Sheath Blight

The control efficacy of *Streptomyces* sp. JCK-6141 strain against rice sheath blight (RSB) was examined *in vivo.* As an experimental plant, the rice cultivar Nakdong (*Oryza sativa* L.) was used.

One day before pathogen inoculation, the Nakdong rice plants were treated by soil drenching with 10 ml of each of the 3-fold and 9-fold dilutions of the *Streptomyces* sp. JCK-6141 strain culture in distilled water. After putting an appropriate amount of Nakdong rice bran treated with the dilutions was put into a 1-L culture bottle and sterilized, followed by evenly inoculating finely ground mycelial mass of cultured *Rhizoctonia solani* into the pots where the rice plants had been grown. After inoculation, incubation was carried out for 3 days in a humid chamber (25°C) and then for 4 days in

an incubator with a relative humidity of 80%. Examination was made of the onset of the disease. As a control, the synthetic chemical fungicide Flutolanil was used at 20 μg/ml or 50 μg/ml concentration. For incidence rate, infected areas in the rice sheath were examined, and the results are summarized in Table 15, below.

TABLE 15

| Treated group | Concentration (fold, pg/ml) | RSB Control (%) |
|---|---|---|
| JCK-6164 Culture broth (CB) | 3-fold | 35.0±5.0 b |
| | 9-fold | 15.0±5.0 c |
| Chemical fungicide (Flutolanil) | 50 μg/ml | 100.0±0.0 a |
| | 20 μg/ml | 100.0±0.0 a |

As shown in Table 15 and FIG. 13, the 3-fold dilution of *Streptomyces* sp. JCK-6141 strain culture exhibited a control activity of 35.0% against rice sheath blight and, although lower in control value than the 3-fold dilution, the 9-fold dilution of the culture exhibited a control activity of 15.0%. These data imply that, although low in control efficacy compared to the synthetic chemical fungicide Flutolanil, *Streptomyces* sp. JCK-6141 strain culture can effectively control rice sheath blight, with a control value of 35.0%.

EXAMPLE 11. *In Vivo* Assay for Controlling Effect of *Streptomyces* sp. JCK-6141 Strain on Wheat Leaf Rust

The control efficacy of *Streptomyces* sp. JCK-6141 strain on wheat leaf rust was examined *in vivo.* As an experimental plant, the wheat cultivar Eunpa (*Triticum aestivum* L.) was used.

One day before pathogen inoculation, the Eunpa wheat plants were treated by soil drenching with 10 ml of each of the 3-fold and 9-fold dilutions of the *Streptomyces* sp. JCK-6141 strain culture in distilled water. Since the pathogen *Puccinia recondite* is an obligate biotroph, uredospores formed on wheat seedlings as a result of passages of the plant in the laboratory were used as an inoculum. A uredospore suspension (spore 0.67 g/L) was prepared and sprayed over the wheat seedlings, which were then incubated at 20°C for one day in a humid room and subsequently in an incubator with a relative moisture of 70% to induce the onset of the disease. As a control, the synthetic chemical fungicide Flutolanil was used at 20 μg/ml or 50 μg/ml concentration. For incidence rate, infected areas in the rice sheath were examined 7 days after inoculation, and the results are summarized in Table 16, below.

TABLE 16

| Treated group | Concentration (fold, μg/ml) | RSB Control (%) |
|---|---|---|
| JCK-6164 culture broth (CB) | 3-fold | 80.0±0.0 b |
| | 9-fold | 13.3±6.7 c |
| Chemical fungicide (Flutolanil) | 50 μg/ml | 100.0±0.0 a |
| | 20 μg/ml | 73.3±6.7 b |

As shown in Table 16 and FIG. 14, the 3-fold dilution of *Streptomyces* sp. JCK-6141 strain culture exhibited a control efficacy of 80.0% against wheat leaf rust and, although lower in control value than the 3-fold dilution, the 9-fold dilution of the culture exhibited a control activity of 13.3%. These data imply that, although low in control efficacy compared to the synthetic chemical fungicide Flutolanil, *Streptomyces* sp. JCK-6141 strain culture effectively controls wheat leaf rust, with a control value of 80.0%.

EXAMPLE 12. *In Vivo* Assay for Controlling Effect of Streptomyces sp. JCK-6141 Strain on Fusarium Head Blight

The control efficacy of *Streptomyces* sp. JCK-6141 strain on Fusarium head blight was examined *in vivo.* As an experimental plant, the wheat cultivar Eunpa (*Triticum aestivum* L.) was used.

First, *Streptomyces* sp. JCK-6141 strain was inoculated into a sterile TSB broth and then cultured at 30°C for 3 days with shaking at 150 rpm, to prepare a culture. Each of the 5- and 20-fold dilutions of the *Streptomyces* sp. JCK-6141 strain culture in distilled water was sprayed over ears of wheat, then left for 2 hours. The plants were inoculated with a spore suspension of *Fusarium graminearum,* which is a pathogen causing Fusarium head blight, and the entire pot was covered with a vinyl sheet to maintain humidity for 3 days. The sample was applied to the plants when the ears

flowered. As a positive control, a synthetic pesticide Almuri (2000-fold dilution, Syngenta, Switzerland) was applied while Tween-20 (500 μg/ml) was used as a negative control. Ten ears of wheat were treated three times as a basic unit. The disease severity and incidence rate were examined 2 weeks after inoculation, and the results are summarized in Table 17 below.

TABLE 17

| Inhibitor | Conc. | Disease severity index (%) | Incidence rate (%) | FHB Index (%) | Control (%) |
|---|---|---|---|---|---|
| Almuri | 2000-fold | 17.0 | 75.1 | 12.8 | 86.3 |
| JCK-6141 culture | 5-fold | 49.2 | 100.0 | 49.2 | 47.2 |
| | 20-fold | 67.1 | 100.0 | 67.1 | 28.0 |
| Inhibitor-free control | - | 93.2 | 100.0 | 93.2 | 0.0 |

As shown in Table 17 and FIG. 15, the 5-fold dilution of *Streptomyces* sp. JCK-6141 strain culture exhibited a control activity of 47.2% against Fusarium head blight and, although lower in control value than the 3-fold dilution, the 20-fold dilution of the culture exhibited a control value of 28.0%. These data imply that *Streptomyces* sp. JCK-6141 strain culture, although low in control effect compared to the synthetic chemical fungicide Almuri, is effective for controlling Fusarium head blight, with a control effect amounting to 47.2%.

EXAMPLE 13. *In Vivo* Assay for Controlling Effect of Streptomyces sp. JCK-6141 Strain on Dollar Spot Disease

The control efficacy of *Streptomyces* sp. JCK-6141 strain against dollar spot disease was evaluated *in vivo.*

13-1. Plant preparation

As an experimental plant, seeds of creeping bentgrass (*Agrostis palustris*), which is a type of cool-season grass, were used. In a pot with a diameter of 7 cm and a height of 6 cm, sterilized sand and horticultural gardening soil were mixed at a ratio of 3:1. The seeds were sown and grown for 30 days before use in experiments.

13-2. Sample treatment

After diluting *Streptomyces* sp. JCK-6141 strain culture 3- and 9-fold with distilled water, Tween-20 (250 ug/ml) was added thereto, and then 10 ml per pot was applied by soil drenching 1 day before inoculation with the pathogen. An untreated group was treated with Tween-20 (250 ug/ml) solution while as a control agent, a Horikuo (active ingredient tebuconazole, 25% by weight) emulsion, commercially available for prevention of grass fungus, was diluted 2000-fold and applied by soil drenching to the pots in an amount of 10 ml per pot one day before inoculation. The pathogen *Sclerotinia homoeocarpa* was cultured on PDA (potato dextrose agar) plates for 5 days. The colonies thus formed were cut into pieces, each 1 cm wide and 1 cm long. For use as an inoculum, five pieces were added into each wheat bran-rice husk medium (36 g of wheat bran, 6 g of rice husk, and 40 ml of distilled water in 1 L Erlenmeyer flask) and then cultured for 7 days in a 25°C incubator. After incubation, 400 ml of sterile water (including 200 μg/ml of Streptomycin sulfate) was added. The mixture was ground using a blender and then applied by soil drenching with 4 ml per pot. The treated turfgrass was placed under 25°C and 95% relative humidity to develop the disease for 7 days. The disease severity was examined, and the results are summarized in Table 18, below.

TABLE 18

| Treated group | Concentration (fold) | Control value (%) |
|---|---|---|
| CB (dilution in distilled water) | 3-fold | 76.3±16.5 |
| | 9-fold | 51.5±11.0 |
| Horikuo | 2000-fold | 89.3±5.3 |

As shown in Table 18 and FIGS. 16A and 16B, the 3-fold dilution of *Streptomyces* sp. JCK-6141 strain culture exhibited a control activity of 76% against dollar spot disease, and the 9-fold diluted culture exhibited a control activity of 76%. These data imply that the *Streptomyces* sp. JCK-6141 strain culture, although low in control effect compared to the synthetic chemical fungicide Horikuo, is effective for controlling dollar spot disease, with a control effect amounting to 76%.

## Industrial Applicability

The present disclosure relates to a composition including a culture of *Streptomyces* sp. JCK-6141 or an extract from the culture for controlling plant diseases, a method for producing the same, and a method for controlling plant diseases and, more particularly, to a controlling efficacy identified in a culture of *Streptomyces* sp. JCK-6141 having an antimicrobial activity or an extract from the culture against phytopathogens.

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

**RECEIPT IN THE CASE OF AN ORIGINAL DEPOSIT**

issued pursuant to Rule 7.1

TO: **Industry Foundation of Chonnam National University**
Industry Foundation of Chonnam National University
77, Yongbong-ro, Buk-gu, Gwangju
Republic of Korea

| I. IDENTIFICATION OF THE MICROORGANISM | |
|---|---|
| Identification reference given by the DEPOSITOR:<br><br>***Streptomyces sp.* JCK-6141** | Accession number given by the INTERNATIONAL DEPOSITARY AUTHORITY:<br><br>**KCTC 14333BP** |
| II. SCIENTIFIC DESCRIPTION AND/OR PROPOSED TAXONOMIC DESIGNATION | |
| The microorganism identified under I above was accompanied by:<br>[ ] a scientific description<br>[ ] a proposed taxonomic designation<br>(Mark with a cross where applicable) | |
| III. RECEIPT AND ACCEPTANCE | |
| This International Depositary Authority accepts the microorganism identified under I above, which was received by it on **October 14, 2020.** | |
| IV. RECEIPT OF REQUEST FOR CONVERSION | |
| The microorganism identified under I above was received by this International Depositary Authority on and a request to convert the original deposit to a deposit under the Budapest Treaty was received by it on | |
| V. INTERNATIONAL DEPOSITARY AUTHORITY | |
| Name: **Korean Collection for Type Cultures**<br><br>Address: Korea Research Institute of Bioscience and Biotechnology (KRIBB)<br>181, Ipsin-gil, Jeongeup-si, Jeollabuk-do 56212<br>Republic of Korea | Signature(s) of person(s) having the power to represent the International Depositary Authority or of authorized official(s):<br><br>KIM, Song-Gun, Director<br>Date: **October 14, 2020** |

Form BP/4 (KCTC Form 17) sole page

## Claims

1. A *Streptomyces* sp. JCK-6141 strain, deposited with accession number KCTC 14333BP, having antimicrobial activity.

2. A composition for controlling plant diseases, comprising a culture of a *Streptomyces* sp. JCK-6141 strain deposited with accession number KCTC 14333BP or an extract from the culture.

3. The composition of claim 2, wherein the plant disease is caused by at least one phytopathogen selected from the group consisting of a fungus and an oomycete.

4. The composition of claim 3, wherein the fungus is at least one species selected from the group consisting of *Athelida rolfsii, Botryosphaeria dothidea, Botrytis cinerea, Colletotrichum caudatum, Colletotrichum cocodes, Colletotrichum horii, Curvularia lunata, Endothia parasitica, Fusarium fujikuroi, Fusarium graminearum, Fusarium oxysporum* f. sp. *raphani, Fusarium oxysporum* f. sp. *lycopersici, Fusarium oxysporum* f. sp. *cucumerinum, Gaeumannomyces graminis, Magnaporthiopsis poae, Magnapothe oryzae, Pseudocercospora circumcissa, Raffaelea quercus-mongolicae, Rhizoctonia solani, Rhizoctonia cerealis, Sclerotinia homoeocarpa,* and *Valsa cerasperma.*

5. The composition of claim 3, wherein the fungus is *Puccinia recondita.*

6. The composition of claim 3, wherein the oomycete is at least one species selected from the group consisting of *Phytophthora cactorum, Phytophthora cambivora, Phytophthora capsici, Phytophthora cinnamomi, Pythium graminicola,* and *Pythium ultimum.*

7. The composition of claim 2, wherein the extract from the culture comprises at least one selected from the group consisting of reveromycin E, reveromycin E 1-methyl ester, and a reveromycin E derivative.

8. A method for preparation of a composition for controlling a plant disease, comprising a step of culturing a *Streptomyces* sp. JCK-6141 strain deposited with accession number KCTC 14333BP to afford a culture.

9. The method of claim 8, wherein the plant disease is caused by at least one phytopathogen selected from the group consisting of a fungus and an oomycete.

10. The method of claim 9, wherein the fungus is at least one species selected from the group consisting of *Athelida rolfsii, Botryosphaeria dothidea, Botrytis cinerea, Colletotrichum caudatum, Colletotrichum cocodes, Colletotrichum horii, Curvularia lunata, Endothia parasitica, Fusarium fujikuroi, Fusarium graminearum, Fusarium oxysporum* f. sp. *raphani, Fusarium oxysporum* f. sp. *lycopersici, Fusarium oxysporum* f. sp. *cucumerinum, Gaeumannomyces graminis, Magnaporthiopsis poae, Magnapothe oryzae, Pseudocercospora circumcissa, Raffaelea quercus-mongolicae, Rhizoctonia solani, Rhizoctonia cerealis, Sclerotinia homoeocarpa,* and *Valsa cerasperma.*

11. The method of claim 9, wherein the fungus is *Puccinia recondita.*

12. The method of claim 9, wherein the oomycete is at least one species selected from the group consisting of *Phytophthora cactorum, Phytophthora cambivora, Phytophthora capsici, Phytophthora cinnamomi, Pythium graminicola,* and *Pythium ultimum.*

13. The method of claim 8, wherein the culturing step comprises the following fractionation steps:

   a first fractionation step of obtaining a first active fraction from the culture;
   a second fractionation step of obtaining a second active fraction from the first active fraction using column chromatography and thin layer chromatography;
   a third fractionation step of obtaining a third active fraction from the second active fraction using high-pressure liquid chromatography;
   a fourth fractionation step of separating a fourth active fraction from the third active fraction using preparative thin layer chromatography; and
   a pure substance identification step of identifying the fourth active fraction by high-performance liquid chromatography.

14. The method of claim 13, wherein the pure substance is at least one selected from the group consisting of reveromycin

E, reveromycin E 1-methyl ester, and a reveromycin E derivative.

**15.** A method for controlling a plant disease, comprising a step of treating a plant with a composition containing a culture of a *Streptomyces* sp. JCK-6141 strain deposited with accession number KCTC 14333BP or an extract from the culture for controlling plant diseases.

**16.** The method of claim 15, wherein the treating step is carried out in a manner selected from the group consisting of soil drenching, soil irrigation, foliar spray, trunk injection, foliage treatment, rhizosphere treatment, and seed treatment.

Fig. 1

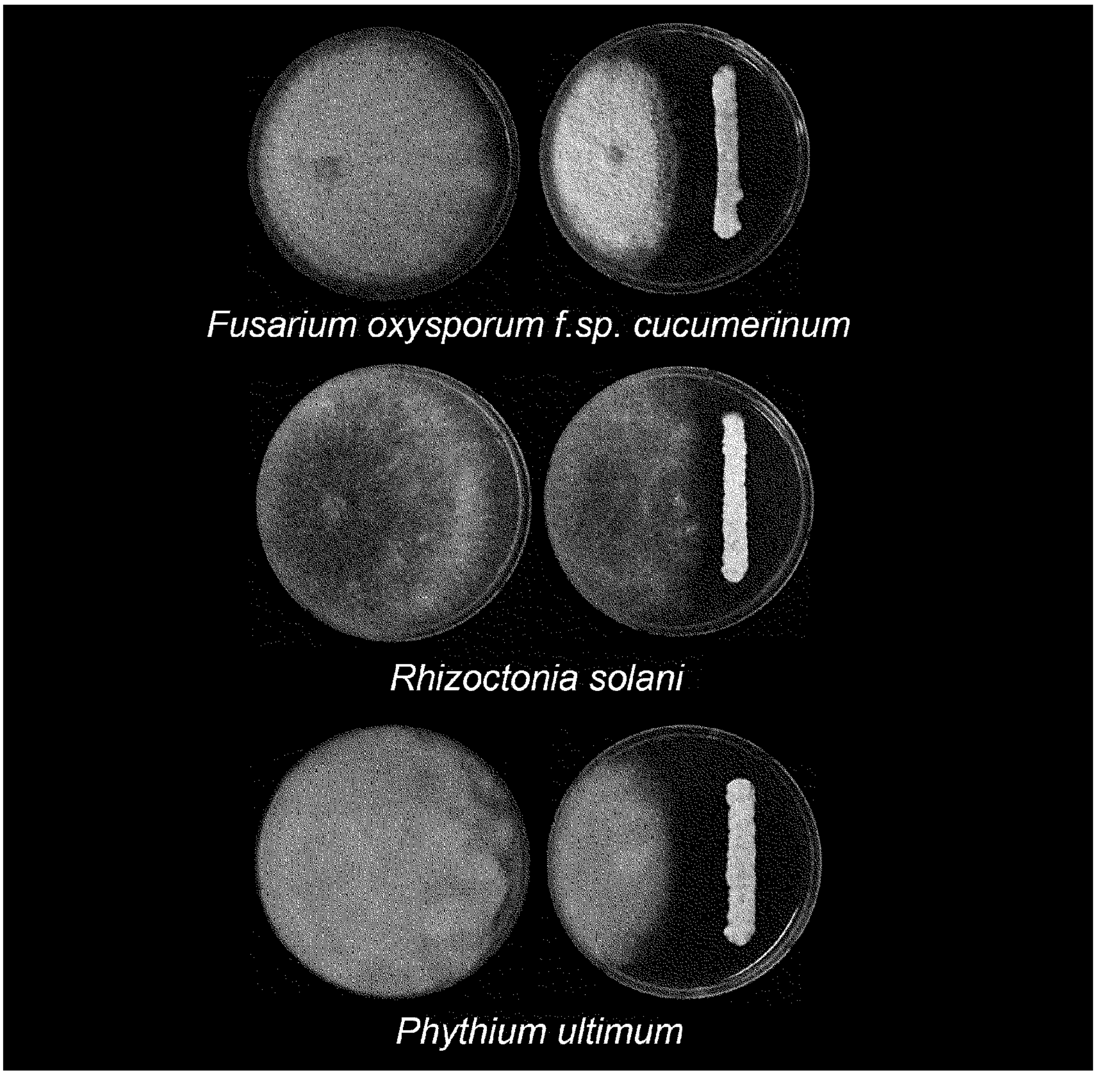
Fusarium oxysporum f.sp. cucumerinum
Rhizoctonia solani
Phythium ultimum

Fig. 2

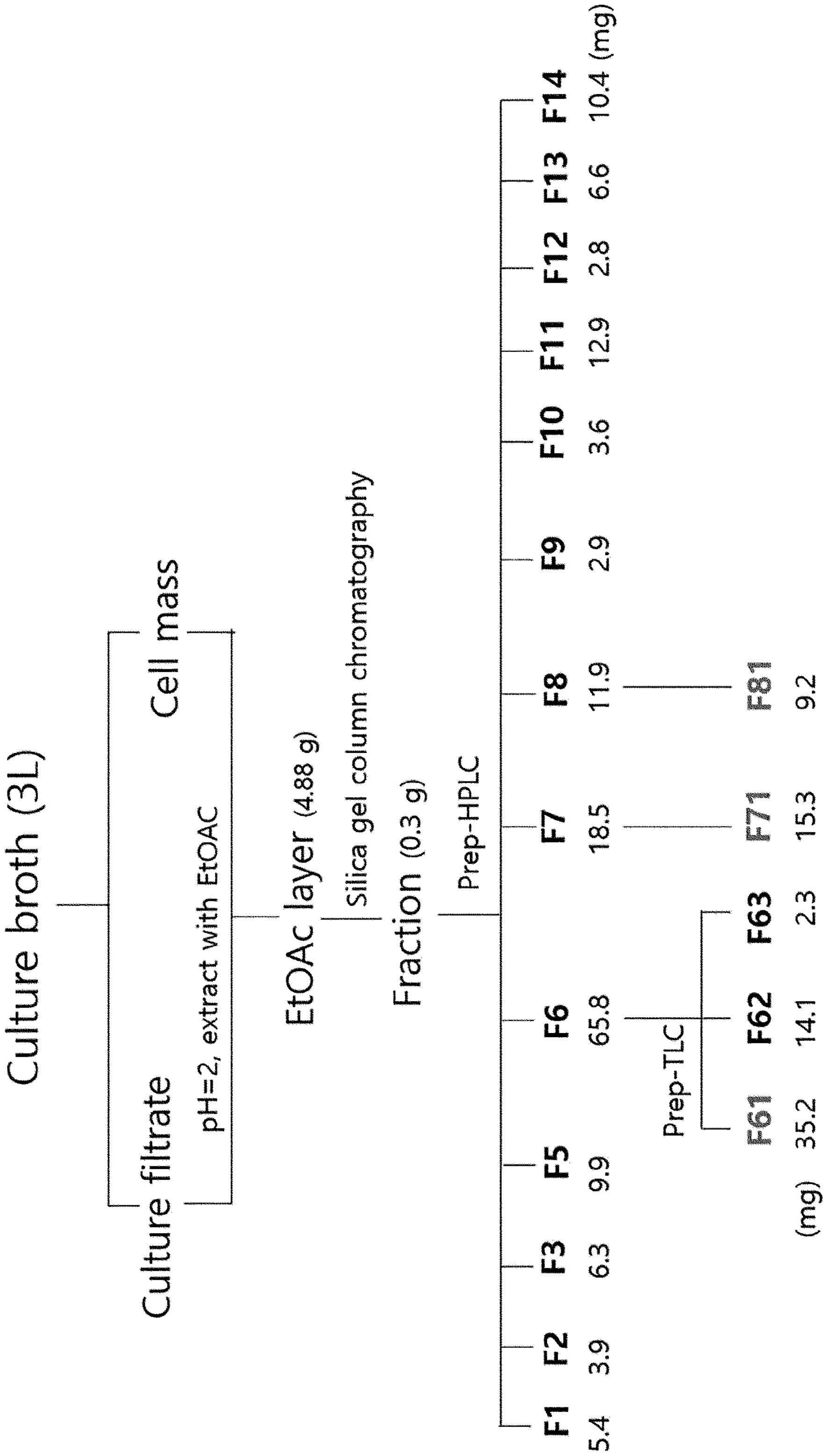
Culture broth (3L)
Culture filtrate
Cell mass
pH=2, extract with EtOAC
EtOAc layer (4.88 g)
Silica gel column chromatography
Fraction (0.3 g)
Prep-HPLC
F1 5.4
F2 3.9
F3 6.3
F5 9.9
F6 65.8
F7 18.5
F8 11.9
F9 2.9
F10 3.6
F11 12.9
F12 2.8
F13 6.6
F14 10.4 (mg)
Prep-TLC
F61 35.2
F62 14.1
F63 2.3
F71 15.3
F81 9.2
(mg)

Fig. 3a

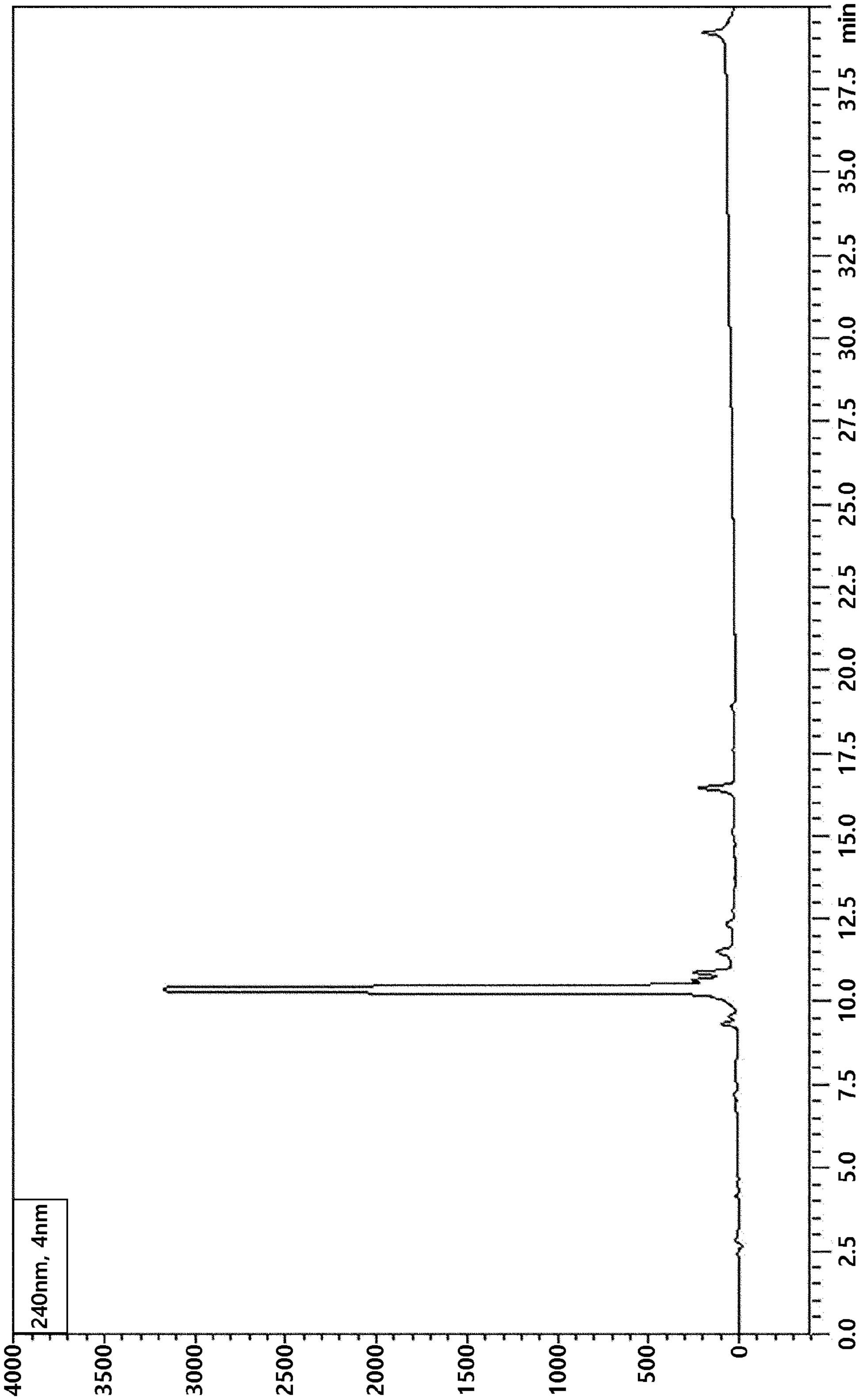
240nm, 4nm
min
0.0
2.5
5.0
7.5
10.0
12.5
15.0
17.5
20.0
22.5
25.0
27.5
30.0
32.5
35.0
37.5
4000
3500
3000
2500
2000
1500
1000
500
0

Fig. 3b

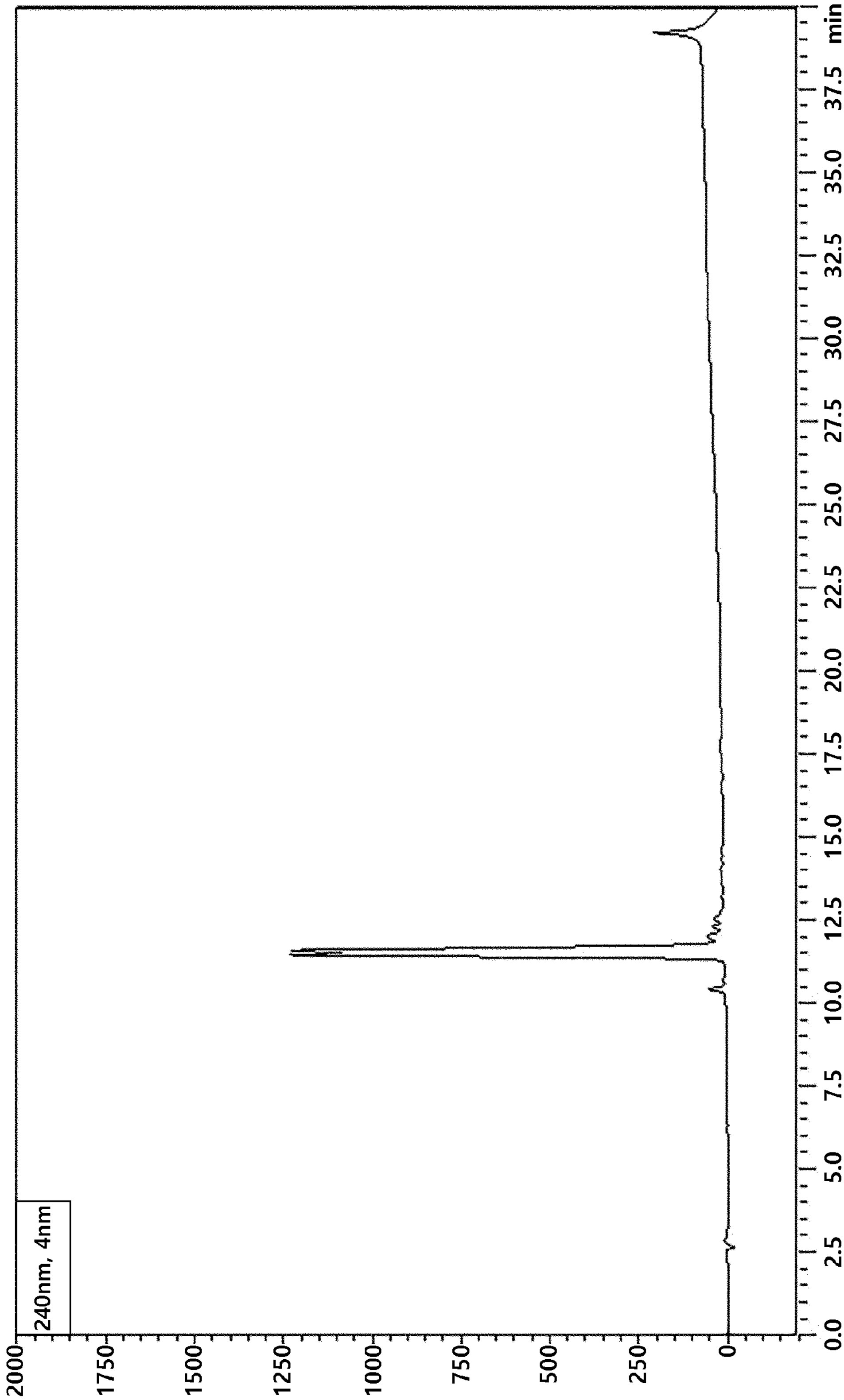
240nm, 4nm
min
0.0
2.5
5.0
7.5
10.0
12.5
15.0
17.5
20.0
22.5
25.0
27.5
30.0
32.5
35.0
37.5
2000
1750
1500
1250
1000
750
500
250
0

Fig. 3c

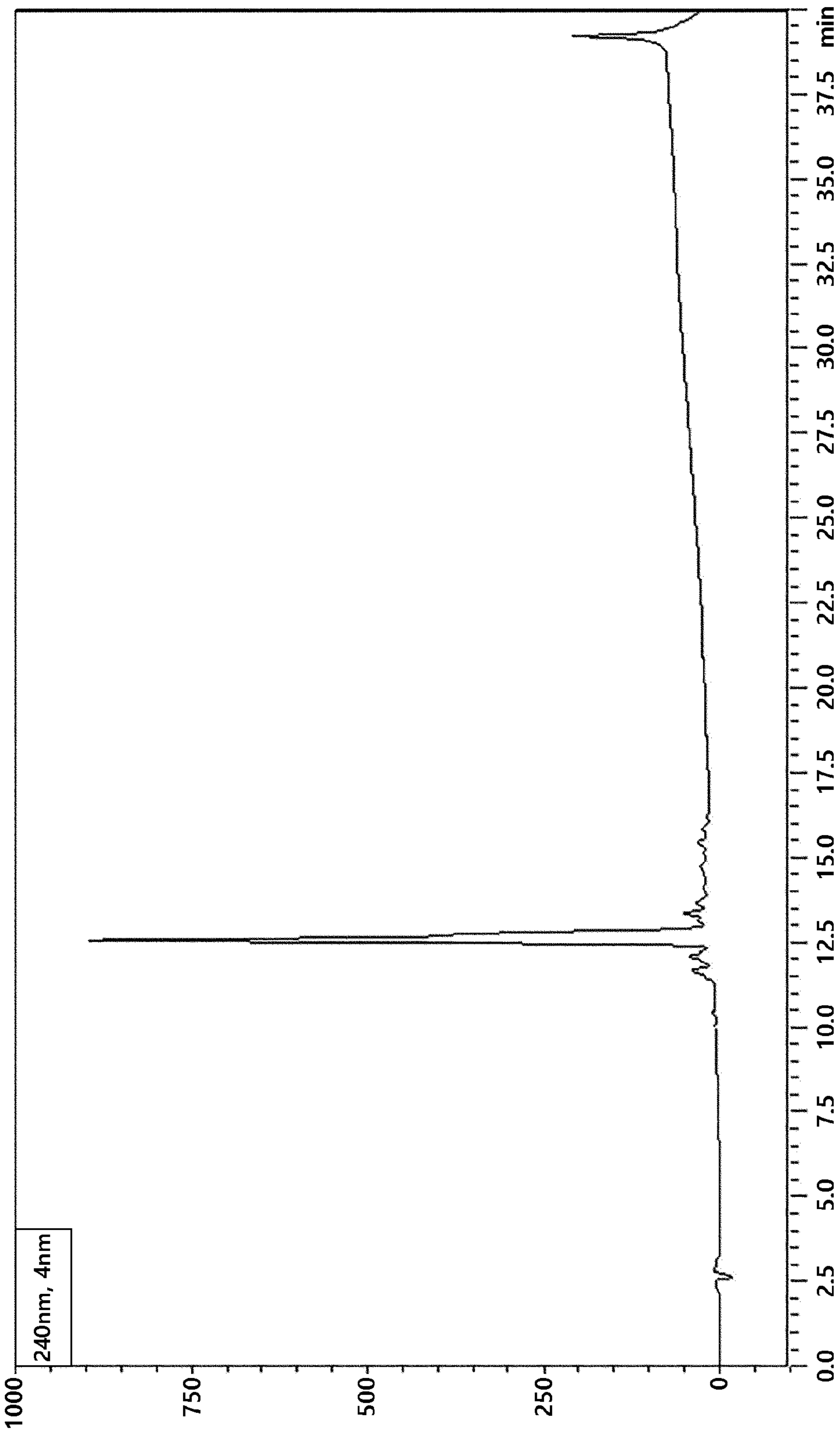
240nm, 4nm
min
0.0
2.5
5.0
7.5
10.0
12.5
15.0
17.5
20.0
22.5
25.0
27.5
30.0
32.5
35.0
37.5
1000
750
500
250
0

Fig. 4a

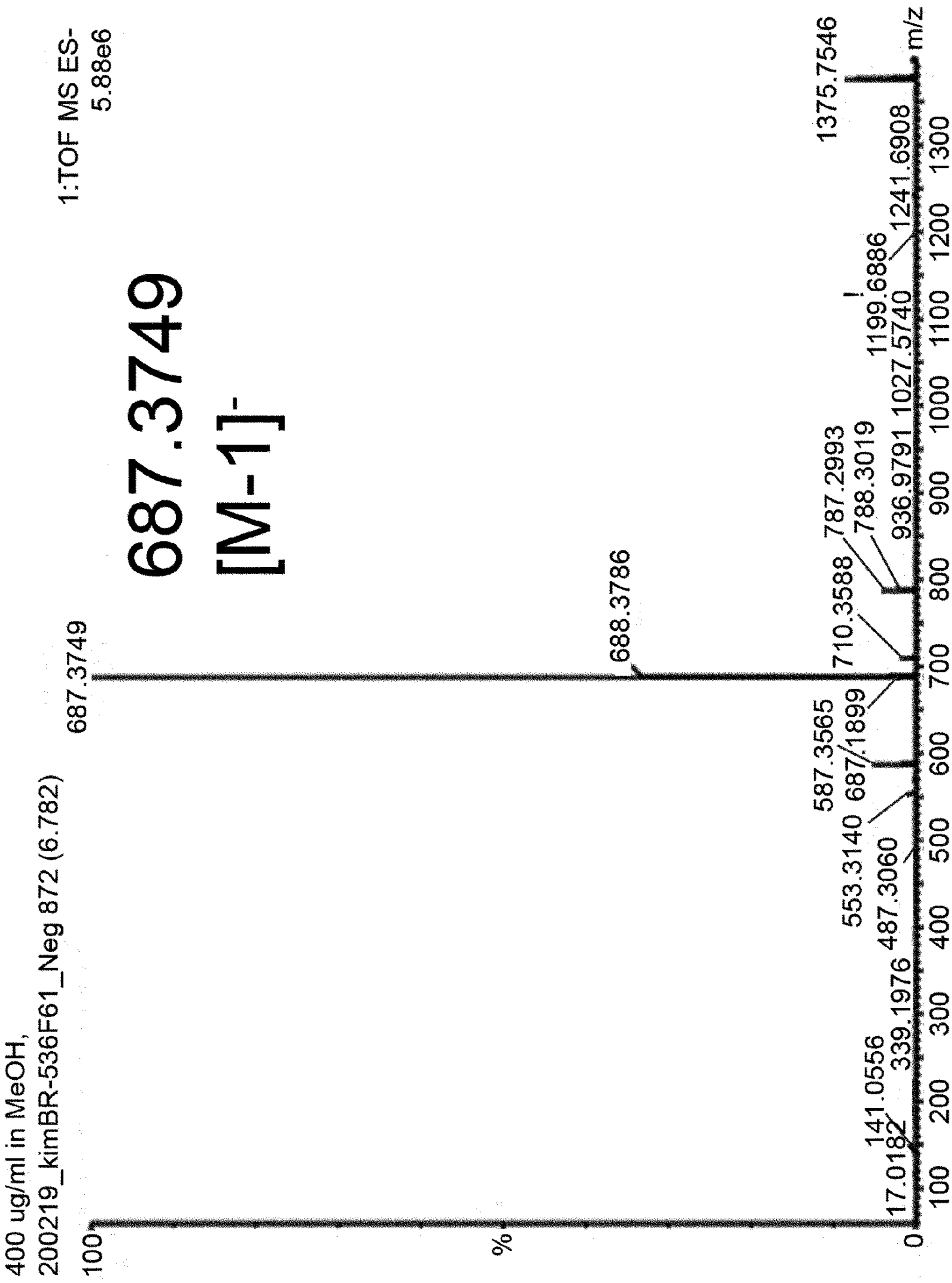
400 ug/ml in MeOH,
200219_kimBR-536F61_Neg 872 (6.782)
1:TOF MS ES-
5.88e6
687.3749
[M-1]-
687.3749
688.3786
710.3588
787.2993
788.3019
936.9791
1027.5740
1199.6886
1241.6908
1375.7546
587.3565
687.1899
553.3140
487.3060
339.1976
141.0556
17.0182
100
%
0
m/z

Fig. 4b

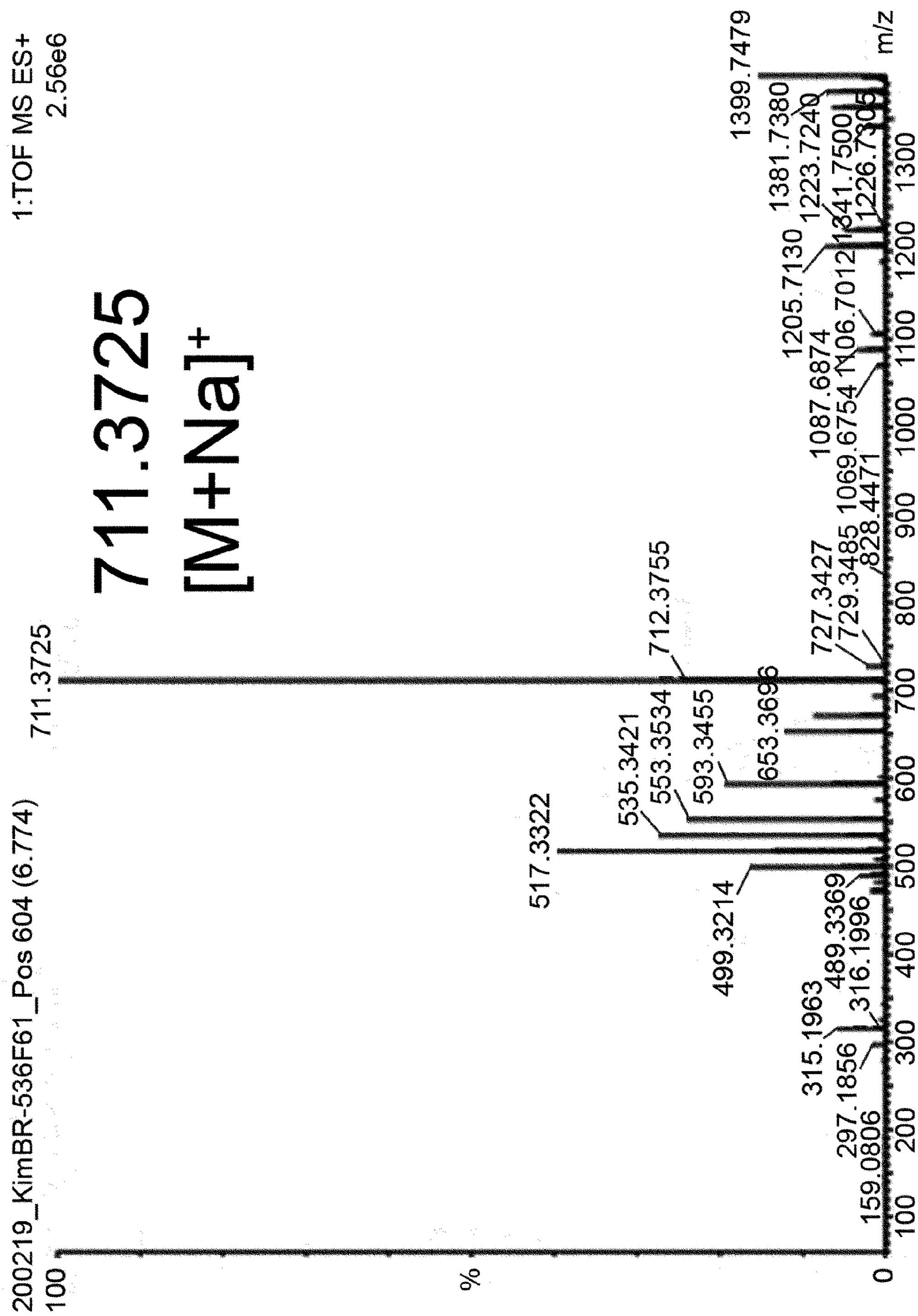
200219_KimBR-536F61_Pos 604 (6.774)
1:TOF MS ES+
2.56e6
711.3725
[M+Na]+
711.3725
712.3755
517.3322
535.3421
553.3534
593.3455
653.3696
499.3214
489.3369
316.1996
315.1963
297.1856
159.0806
727.3427
729.3485
828.4471
1069.6754
1087.6874
1106.7012
1205.7130
1223.7240
1226.7305
1341.7500
1381.7380
1399.7479
100
%
0
m/z
100
200
300
400
500
600
700
800
900
1000
1100
1200
1300

Fig. 5a

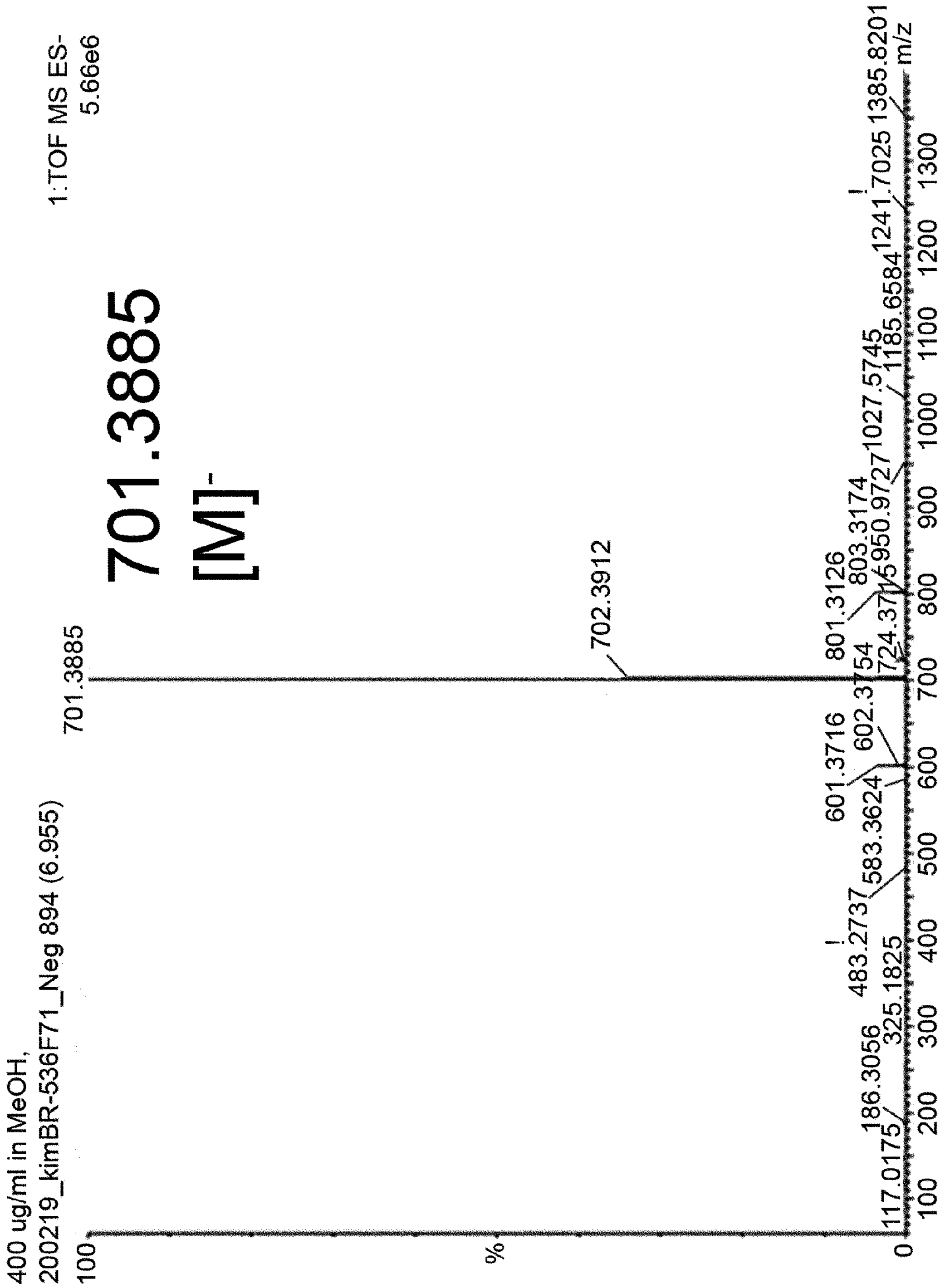
400 ug/ml in MeOH,
200219_kimBR-536F71_Neg 894 (6.955)
1:TOF MS ES-
5.66e6
701.3885
[M]-
701.3885
702.3912
117.0175
186.3056
325.1825
483.2737
583.3624
601.3716
602.3754
724.3715
801.3126
803.3174
950.9727
1027.5745
1185.6584
1241.7025
1385.8201
%
100
0
100
200
300
400
500
600
700
800
900
1000
1100
1200
1300
m/z

Fig. 5b

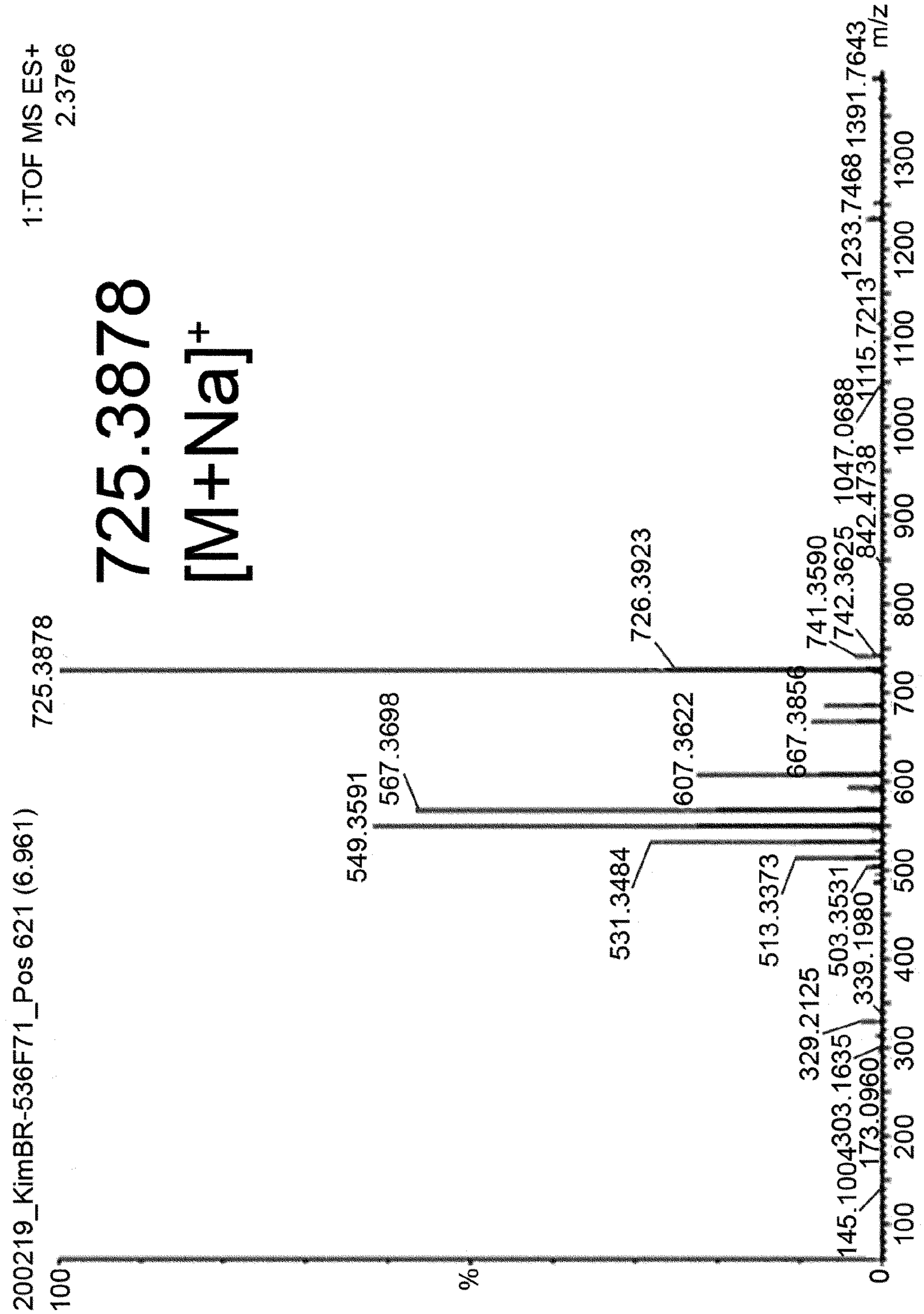
200219_KimBR-536F71_Pos 621 (6.961)
1:TOF MS ES+
2.37e6
725.3878
[M+Na]+
725.3878
567.3698
549.3591
607.3622
531.3484
667.3856
726.3923
741.3590
742.3625
842.4738
1047.0688
1115.7213
1233.7468
1391.7643
513.3373
503.3531
339.1980
329.2125
303.1635
173.0960
145.1004
%
m/z
100
0

Fig. 6a

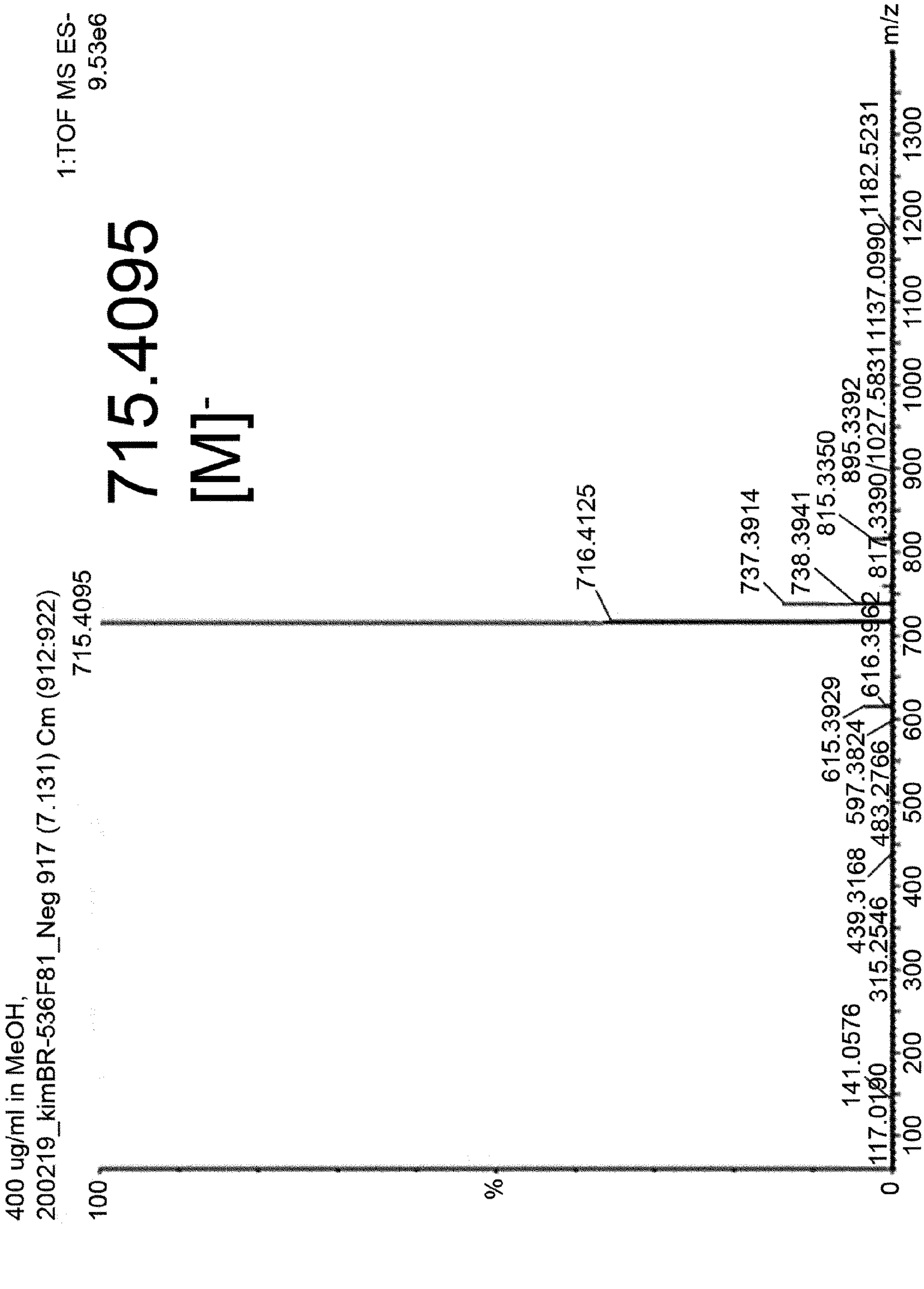
400 ug/ml in MeOH,
200219_kimBR-536F81_Neg 917 (7.131) Cm (912:922)
1:TOF MS ES-
9.53e6
715.4095
[M]-
715.4095
716.4125
737.3914
738.3941
815.3350
895.3392
817.3390/1027.5831
1137.0990
1182.5231
616.3962
615.3929
597.3824
483.2766
439.3168
315.2546
141.0576
117.0190
100
%
0
m/z
100
200
300
400
500
600
700
800
900
1000
1100
1200
1300

Fig. 6b

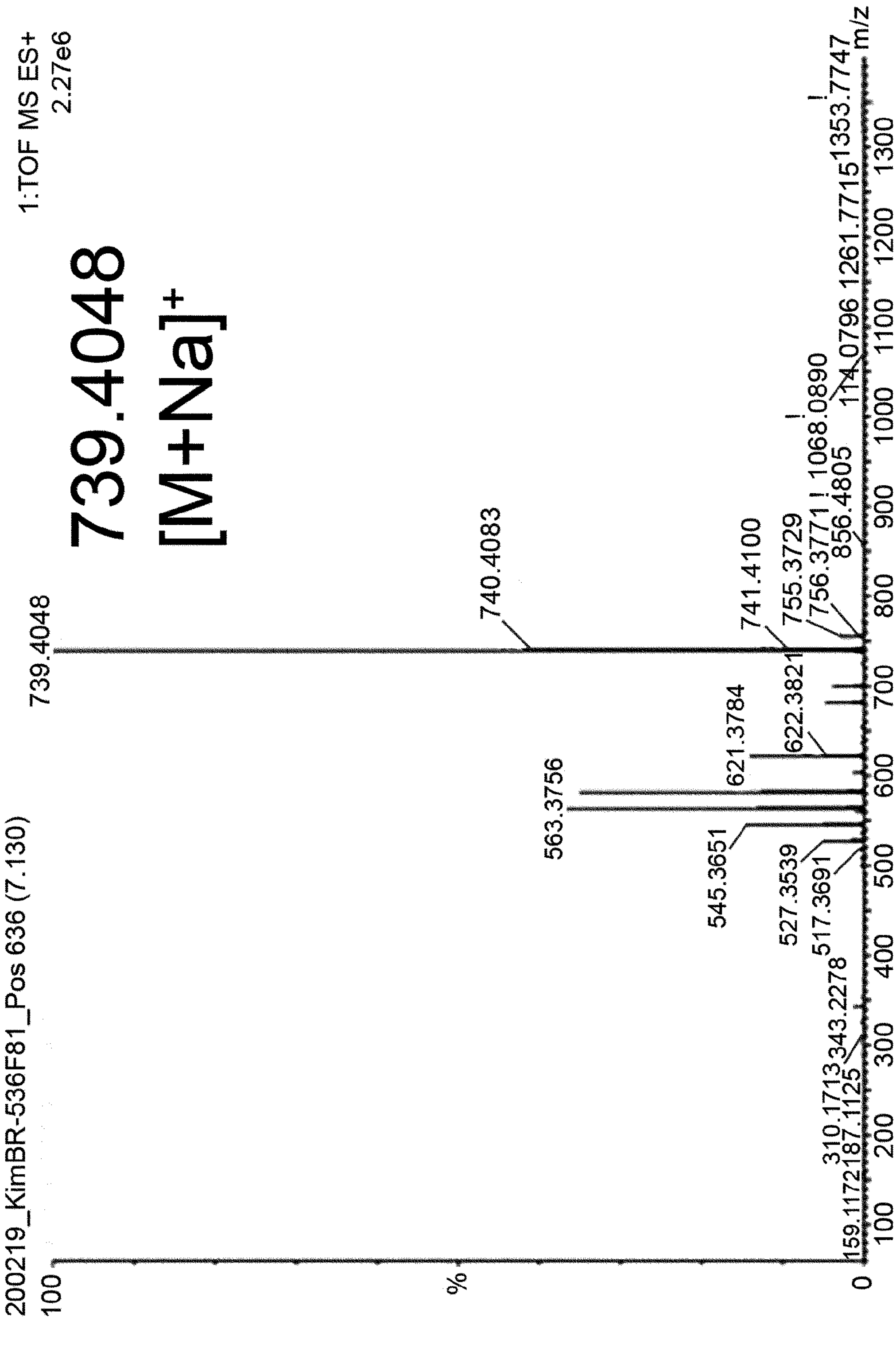
200219_KimBR-536F81_Pos 636 (7.130)
1:TOF MS ES+
2.27e6
739.4048
[M+Na]+
100
%
0
739.4048
740.4083
741.4100
755.3729
756.3771
856.4805
1068.0890
1140796
1261.7715
1353.7747
563.3756
621.3784
622.3821
545.3651
527.3539
517.3691
343.2278
310.1713
187.1125
159.1172
100
200
300
400
500
600
700
800
900
1000
1100
1200
1300
m/z

Fig. 7a

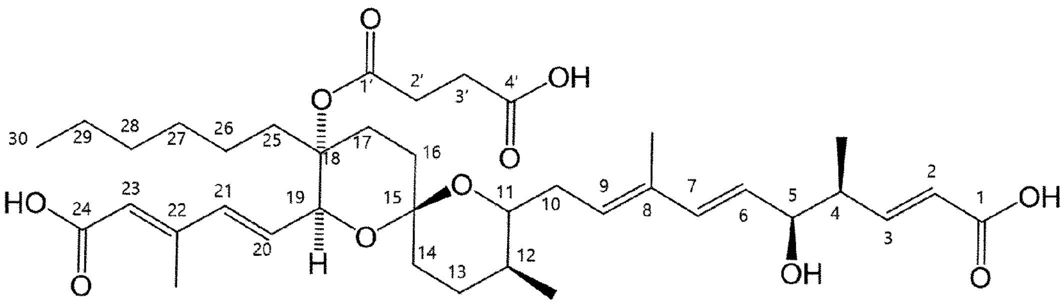

Fig. 7b

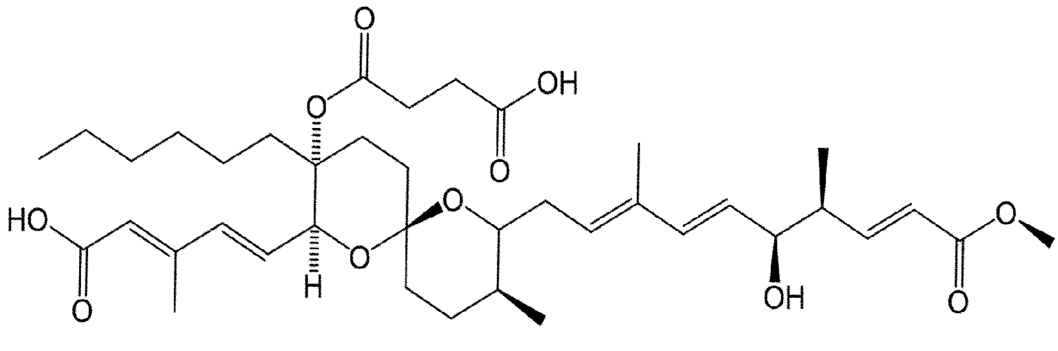

Fig. 8

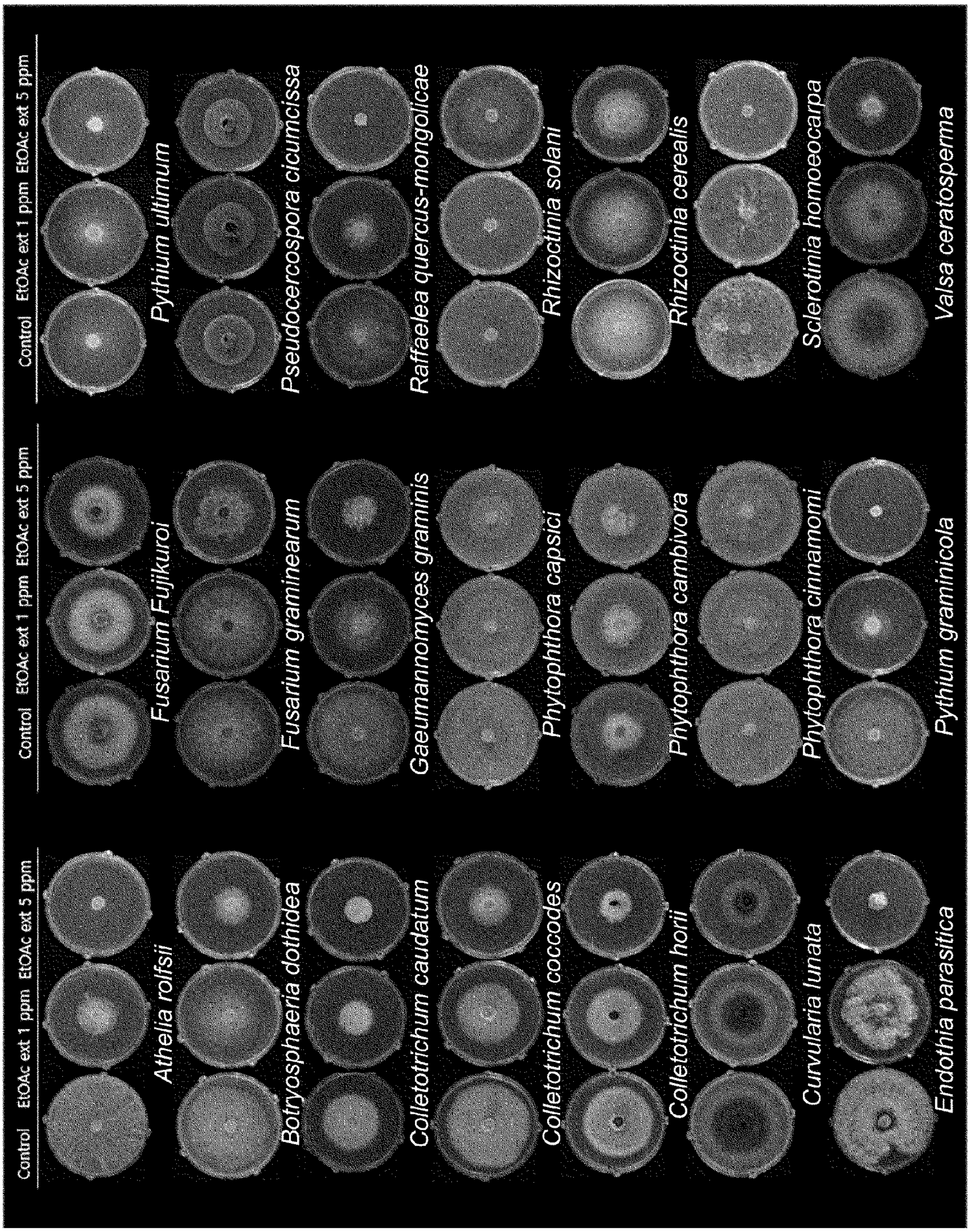
Control
EtOAc ext 1 ppm
EtOAc ext 5 ppm
Pythium ultimum
Pseudocercospora cicumcissa
Raffaelea quercus-mongolicae
Rhizoctinia solani
Rhizoctinia cerealis
Sclerotinia homoeocarpa
Valsa ceratosperma
Fusarium Fujikuroi
Fusarium graminearum
Gaeumannomyces graminis
Phytophthora capsici
Phytophthora cambivora
Phytophthora cinnamomi
Pythium graminicola
Athelia rolfsii
Botryosphaeria dothidea
Colletotrichum caudatum
Colletotrichum coccodes
Colletotrichum horii
Curvularia lunata
Endothia parasitica

Fig. 9a

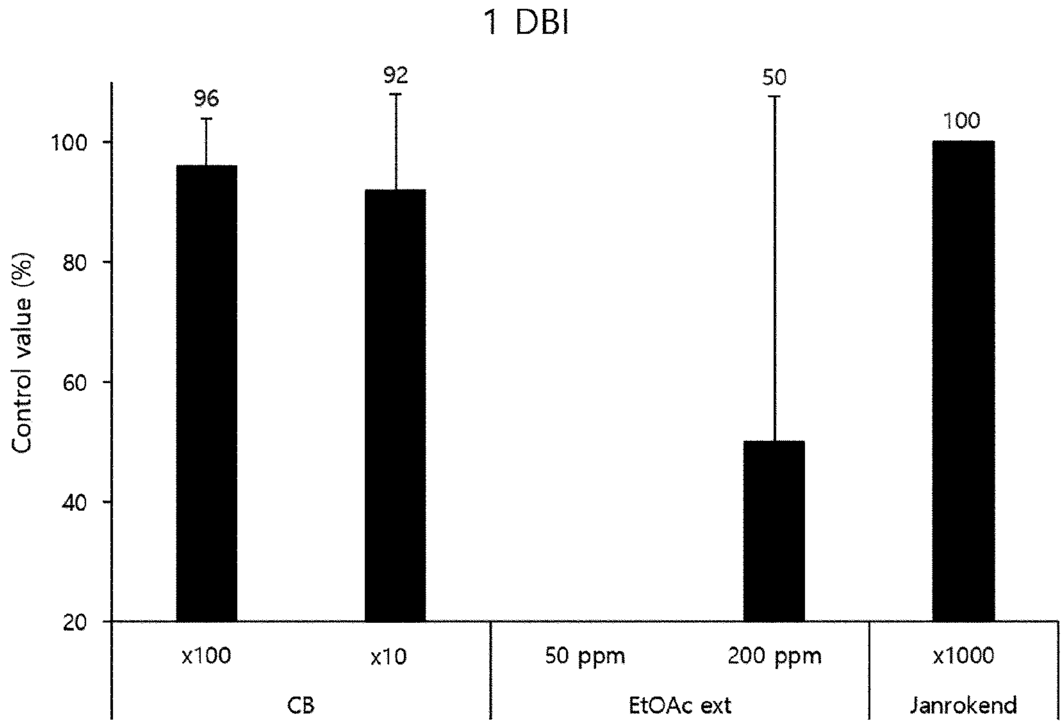
1 DBI
Control value (%)
20
40
60
80
100
96
92
50
100
x100
x10
50 ppm
200 ppm
x1000
CB
EtOAc ext
Janrokend

Fig. 9b

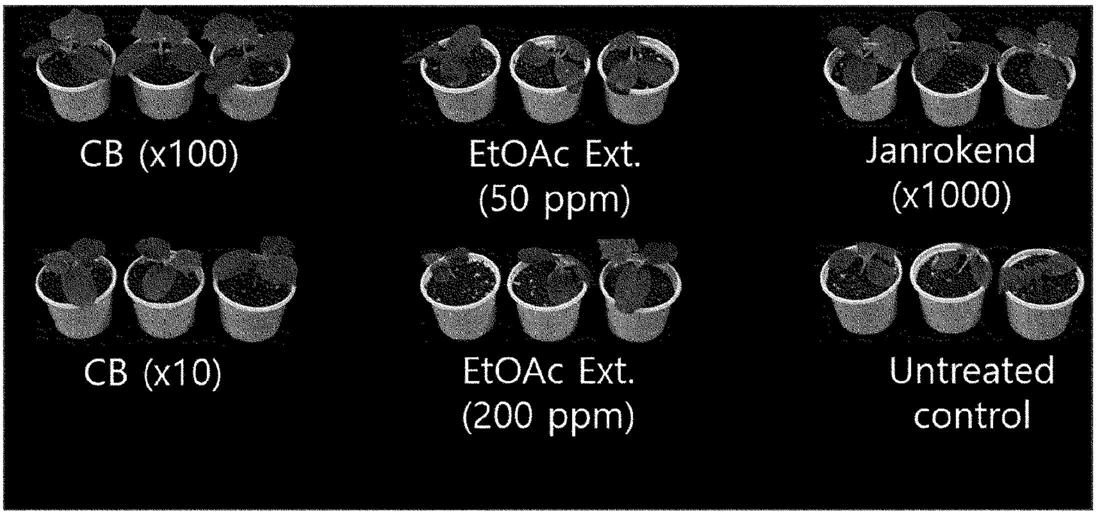
CB (x100)
EtOAc Ext.
(50 ppm)
Janrokend
(x1000)
CB (x10)
EtOAc Ext.
(200 ppm)
Untreated
control

Fig. 10a

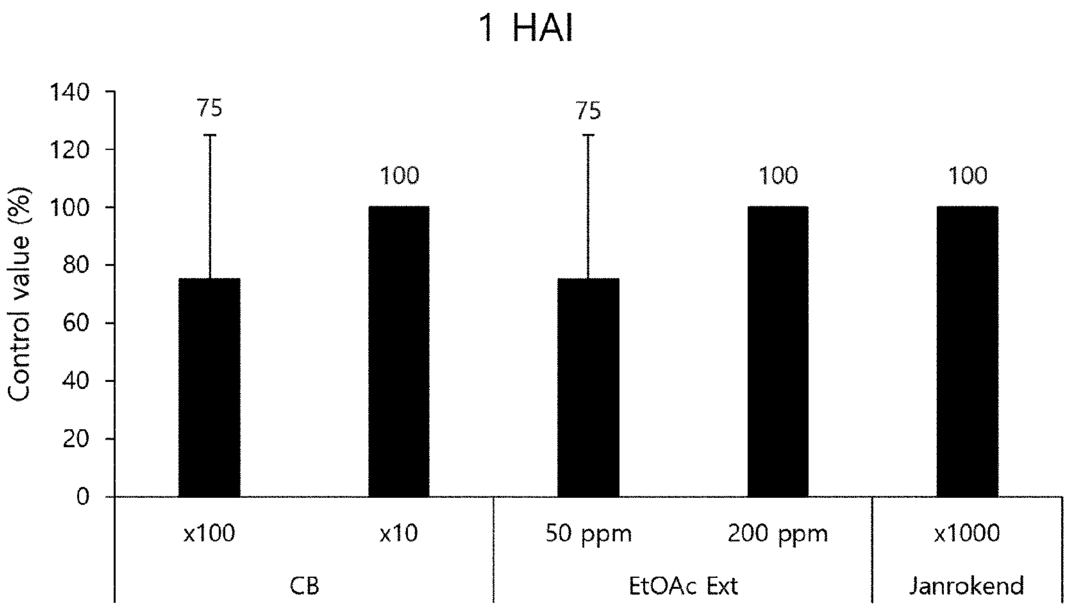


Fig. 10b

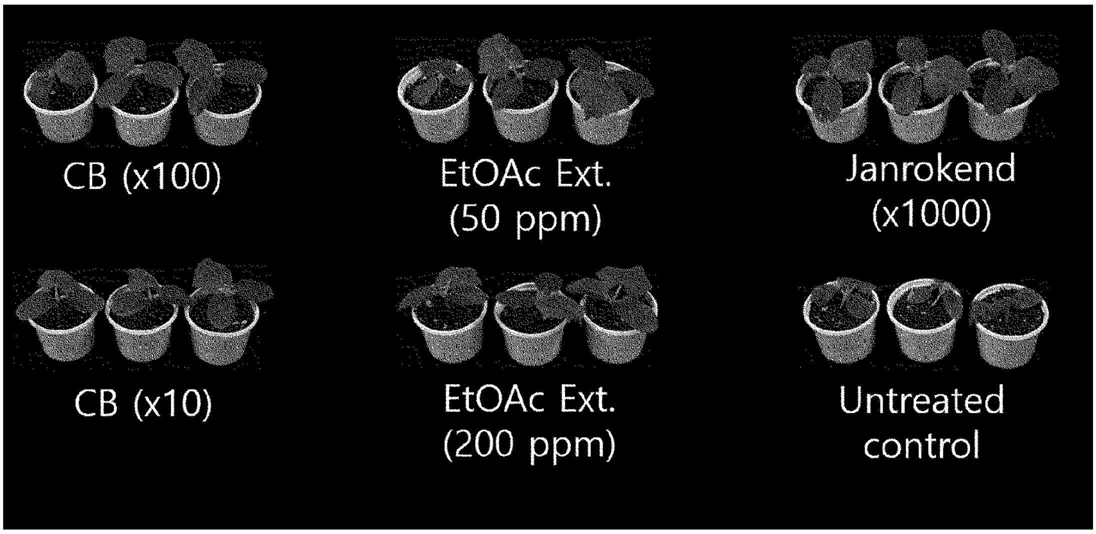

Fig. 11a

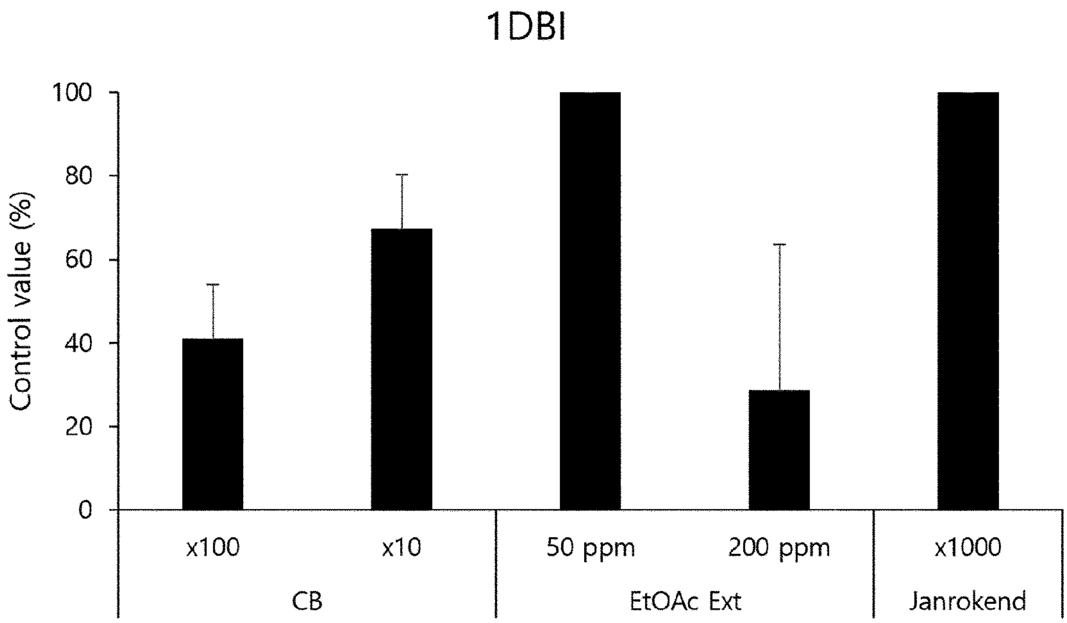


Fig. 11b

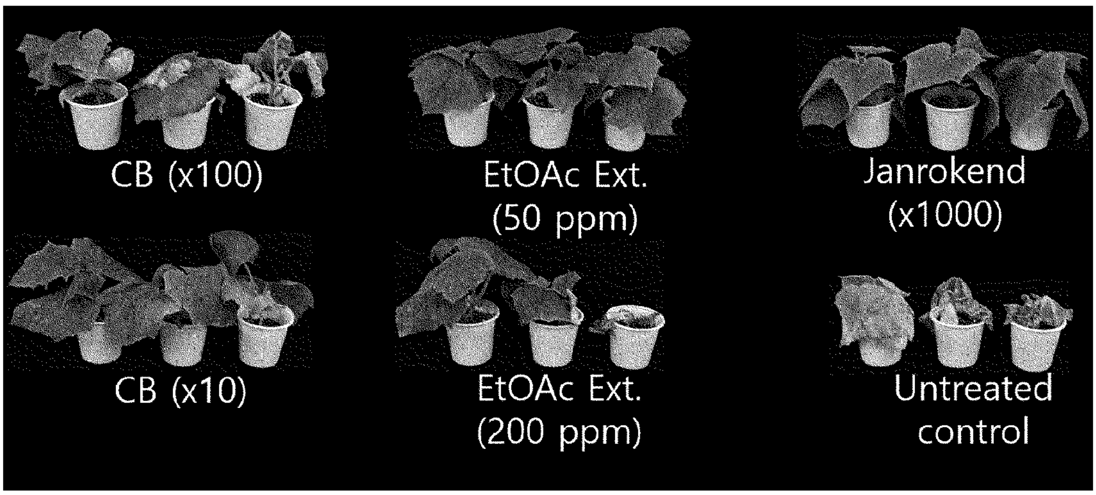

Fig. 12a

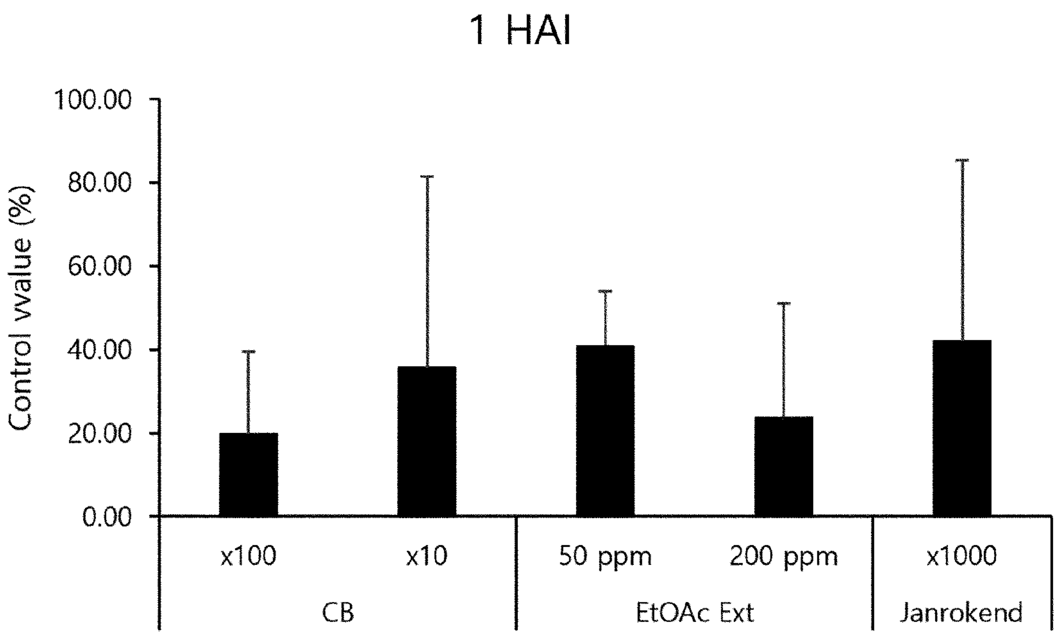


Fig. 12b

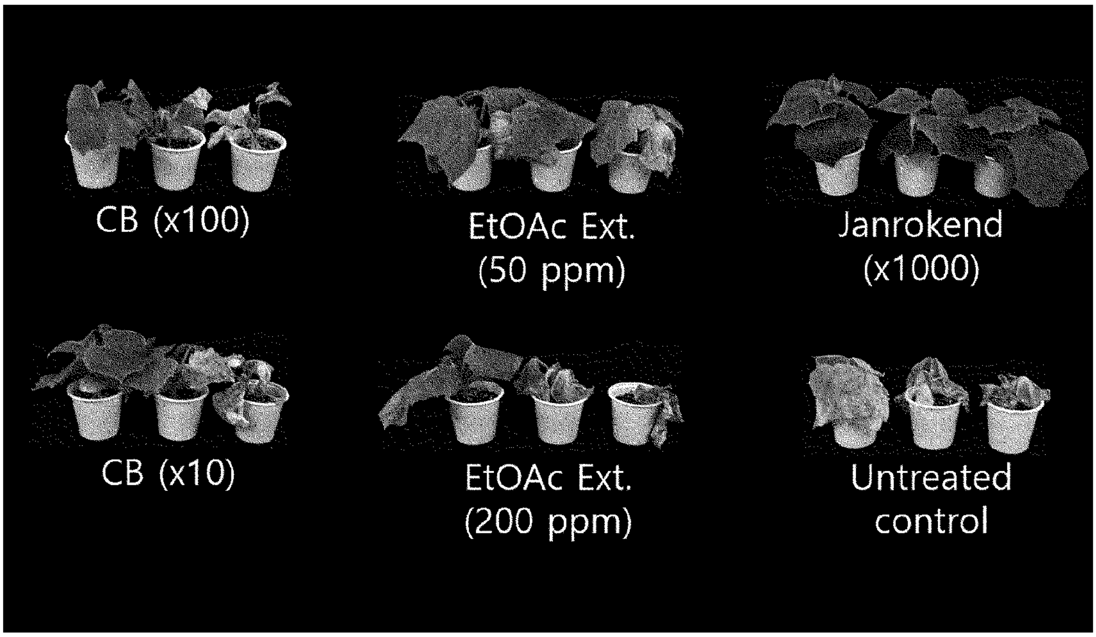

Fig. 13

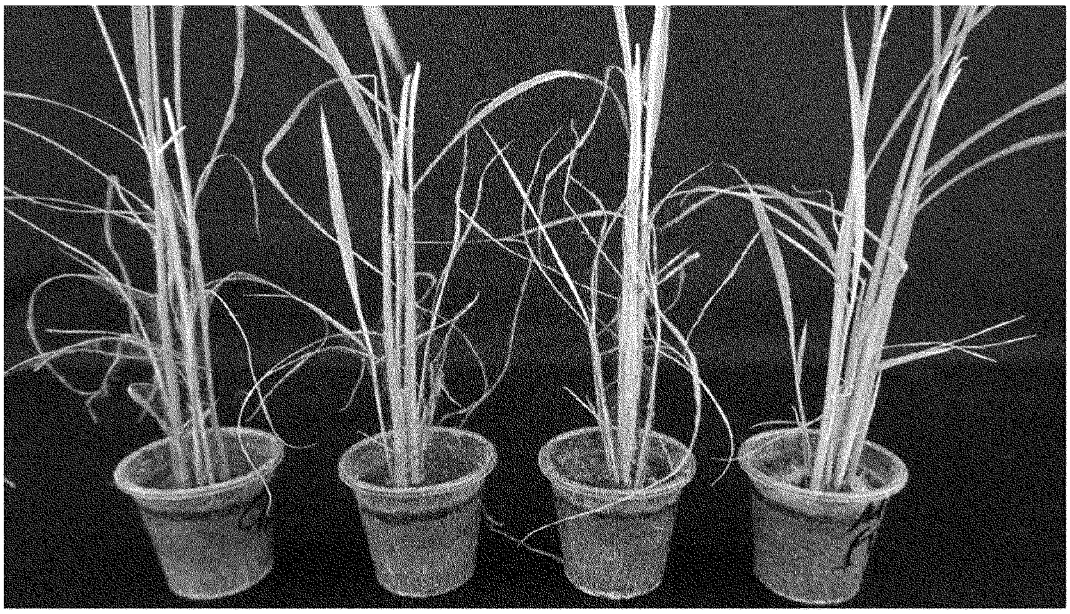

Control CB (x3) CB (x9) Flutolanil (50 ug/ml)

Fig. 14

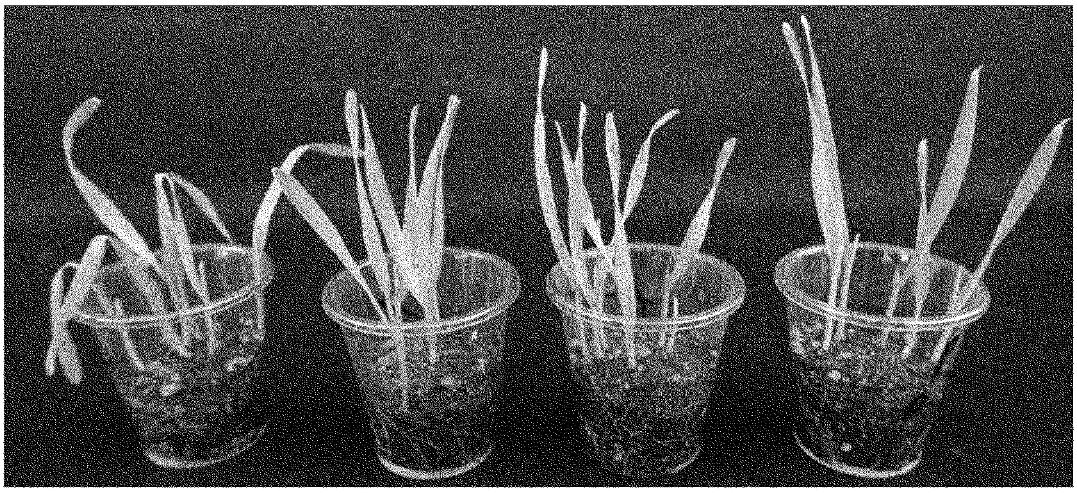

Control CB (x3) CB (x9) Flusilazole (10 ug/ml)

Fig. 15

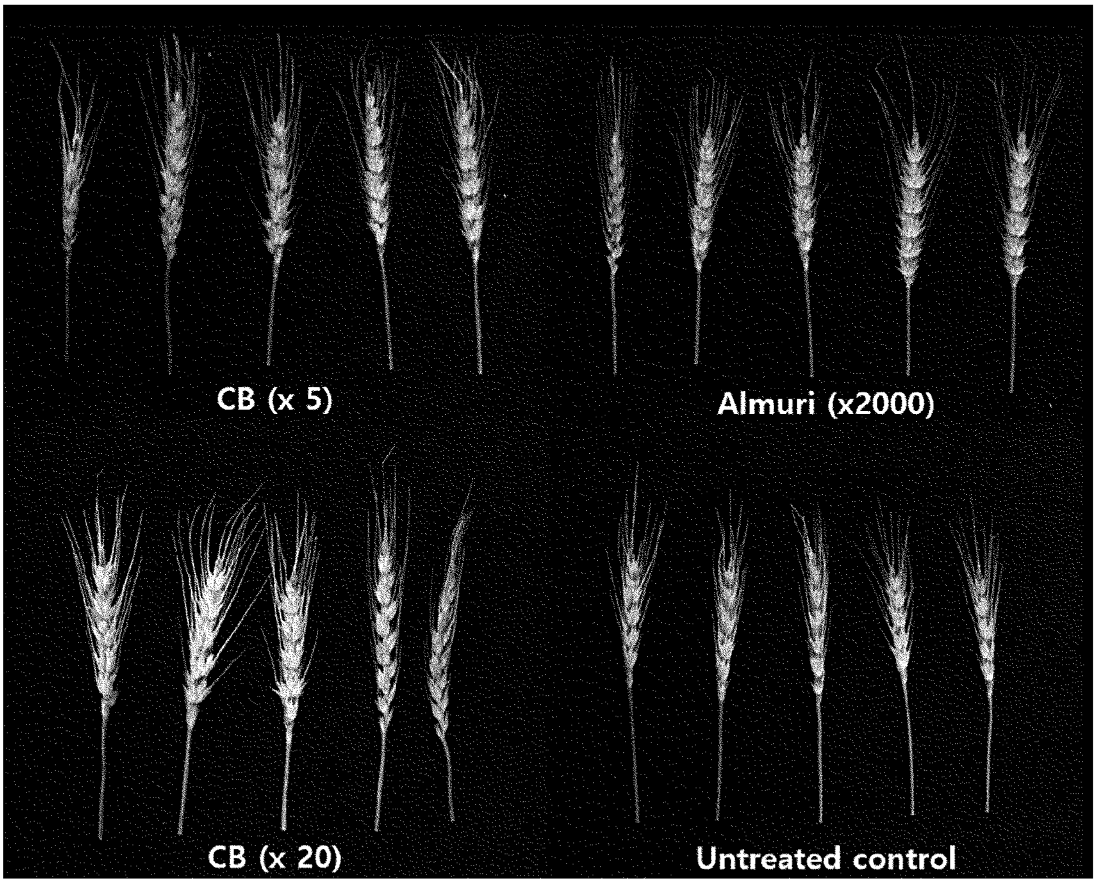

Fig. 16a

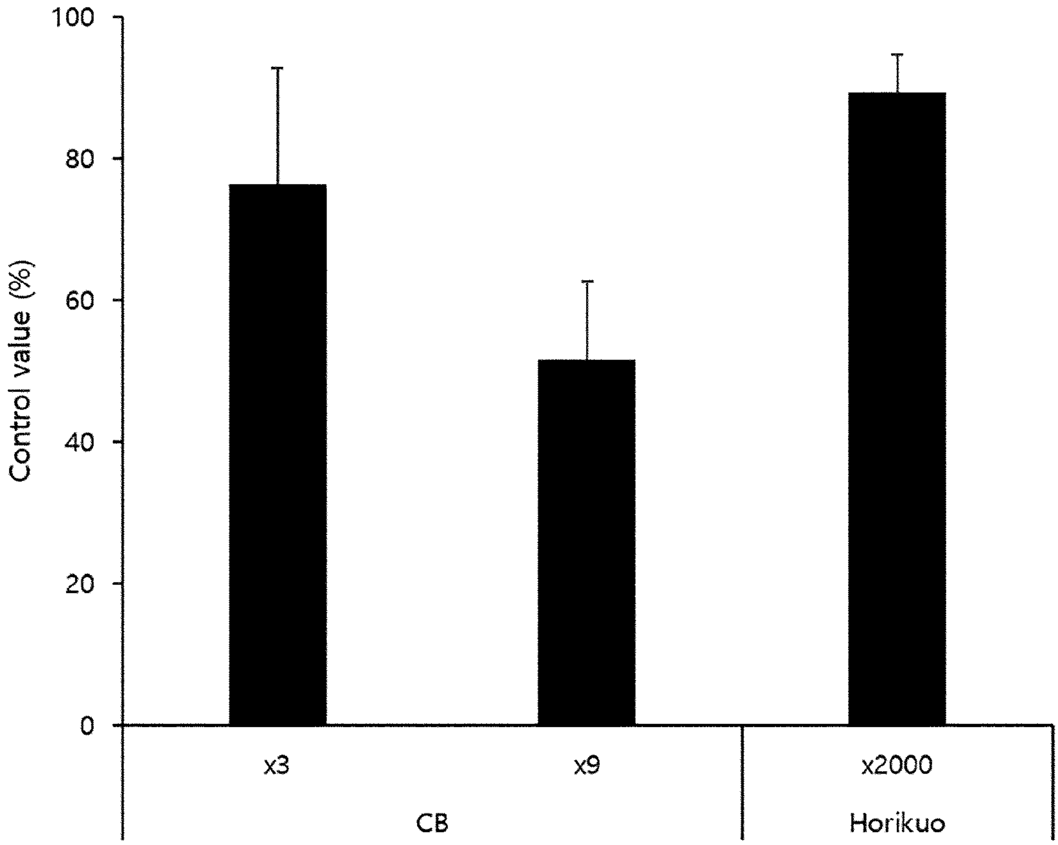

Fig. 16b

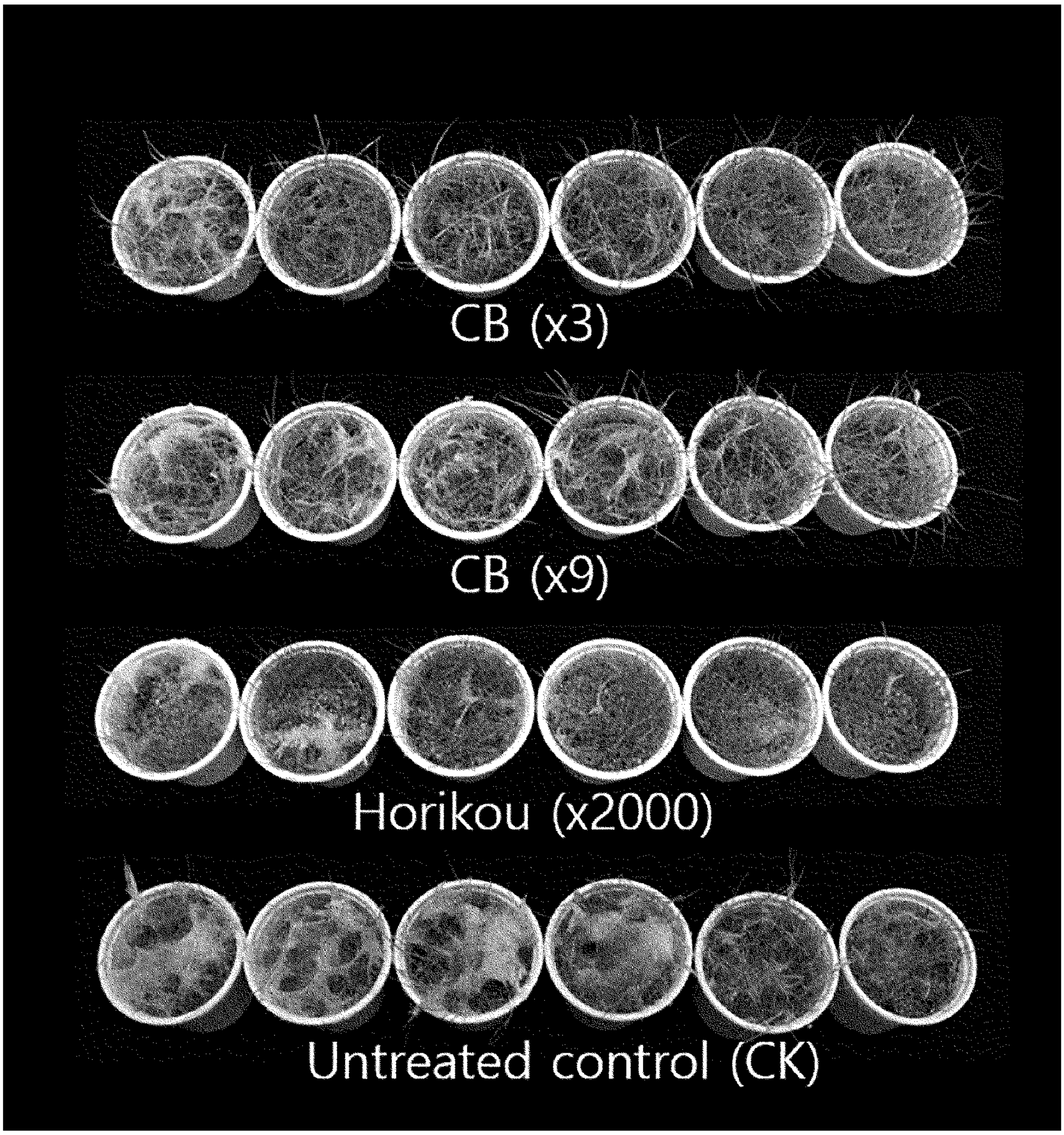

## INTERNATIONAL SEARCH REPORT

International application No.
**PCT/KR2021/016450**

**A. CLASSIFICATION OF SUBJECT MATTER**

**C12N 1/20**(2006.01)i; **A01N 63/28**(2020.01)i; C12R 1/465(2006.01)n; **G01N 30/02**(2006.01)i

According to International Patent Classification (IPC) or to both national classification and IPC

**B. FIELDS SEARCHED**

Minimum documentation searched (classification system followed by classification symbols)

C12N 1/20(2006.01); A01N 63/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 스트렙토마이세스 에스피(Streptomyces sp.), 진균류(fungus), 난균류(oomycete), 식물병 방제(plant disease control), 리베로마이신 E(Reveromycin E)

**C. DOCUMENTS CONSIDERED TO BE RELEVANT**

| Category* | Citation of document, with indication, where appropriate, of the relevant passages | Relevant to claim No. |
|---|---|---|
| X | KR 10-2011-0058015 A (KOREA RESEARCH INSTITUTE OF BIOSCIENCE AND BIOTECHNOLOGY) 01 June 2011 (2011-06-01)<br>See abstract; examples 2-3; claims 1-2 and 5-6; and table 7. | 1-6,8-12,15-16 |
| X | LYU, Ang et al. Reveromycins A and B from Streptomyces sp. 3–10: Antifungal activity against plant pathogenic fungi in vitro and in a strawberry food model system. Frontiers in Microbiology. 2017, vol. 8, 550.<br>See abstract; pages 2-5 and 8-9; tables 1 and 4-5; and figure 2. | 1-4,6-10,12-15 |
| X | US 6280719 B1 (SUH, Hyung-Won) 28 August 2001 (2001-08-28)<br>See abstract; and claims 1-4. | 1-4,6,8-10,12,15-16 |
| A | FREMLIN, Leith et al. Reveromycins revealed: New polyketide spiroketals from Australian marine-derived and terrestrial Streptomyces spp. A case of natural products vs. artifacts. Organic & Biomolecular Chemistry. 2011, vol. 9, pp. 1201–1211.<br>See entire document. | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 March 2022** | **02 March 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No. **PCT/KR2021/016450**

**C. DOCUMENTS CONSIDERED TO BE RELEVANT**

| Category* | Citation of document, with indication, where appropriate, of the relevant passages | Relevant to claim No. |
|---|---|---|
| PX | NGUYEN, Hang Thi Thu et al. Identification and delineation of action mechanism of antifungal agents: Reveromycin E and its new derivative isolated from Streptomyces sp. JCK-6141. Postharvest Biology and Technology. 25 August 2021 (online), vol. 182, 111700. See abstract; pages 2-4; table 2; and figure 2. | 1-4,6-10,12-15 |

Form PCT/ISA/210 (second sheet) (July 2019)

**INTERNATIONAL SEARCH REPORT**
**Information on patent family members**

International application No.
**PCT/KR2021/016450**

| Patent document cited in search report | Publication date (day/month/year) | Patent family member(s) | Publication date (day/month/year) |
|---|---|---|---|
| KR 10-2011-0058015 A | 01 June 2011 | KR 10-1060573 B1 | 31 August 2011 |
| US 6280719 B1 | 28 August 2001 | CN 1246890 A | 08 March 2000 |
| | | EP 1015555 A1 | 05 July 2000 |
| | | JP 2000-513582 A | 17 October 2000 |
| | | JP 3428658 B2 | 22 July 2003 |
| | | KR 10-0197077 B1 | 15 June 1999 |
| | | KR 10-1998-0067506 A | 15 October 1998 |
| | | WO 98-35017 A1 | 13 August 1998 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020200153115
- KR 1020210153374